# EUROPEAN PATENT SPECIFICATION

(11) **EP 0 960 335 B1**
(45) Date of publication and mention of the grant of the patent: **25.05.2005**
(21) Application number: 98903515.9
(22) Date of filing: 15.01.1998
(51) Int. Cl.: G01N 33/53, G01N 33/68

(54) **MODULATORS OF INSULIN RECEPTOR ACTIVITY**
MODULATOREN VON INSULINREZEPTOR-AKTIVITÄT
MODULATEURS DE L'ACTIVITE DU RECEPTEUR D'INSULINE

(30) Priority: 15.01.1997 US 784854; 15.01.1997 US 784855; 15.01.1997 US 784857; 27.03.1997 US 825269; 21.08.1997 US 916088
(43) Date of publication of application: 01.12.1999
(73) Proprietor: Telik, Inc., South San Francisco, CA 94080 (US)
(72) Inventor: KAUVAR, Lawrence, M., San Francisco, CA 04080 (US); SPORTSMAN, Richard, Palo Alto, CA 94306 (US); VILLAR, Hugo, O., Newark, CA 94560 (US); SPEVAK, Wayne, R., Albany, CA 94706 (US); KOHANSKI, Ron, A., New York, NY 10029 (US); SATYAM, Apparao, Freemont, CA 94536 (US); KOEHLER, Ryan, Hayward, CA 94545 (US)
(74) Representative: HOFFMANN - EITLE
(86) International application number: PCT/US1998/000801
(87) International publication number: WO 1998/032017

(56) References cited:
- WO-A-95/23231
- WO-A-96/30762
- S L MACAULAY, M POLITES, M J FRENKEL, D R HEWISH, C W WARD: "Mutagenic structure/function analysis of the cytoplasmic cysteines of the insulin receptor" BIOCHEMICAL JOURNAL, vol. 306, no. 3, 15 March 1995, pages 811-820, XP002088032
- C DESBOIS-MOUTHON, C DANAN, S ANSELEM, M-J BLIVET-VAN EGGELPOEL, C SERT-LANGERON, M GOOSSENS, C BESMOND, J CAPEAU, M CARON: "Severe Resistance to Insulin and Insulin-Like Growth-Factor-I in Cells From a Patient With Leprechaunism as a Result of Two Mutations in the Tyrosine Kinase Domain of the Insulin Receptor" METABOLISM, CLINICAL AND EXPERIMENTAL, vol. 45, no. 12, December 1996, pages 1493-1500, XP002088033
- D E MOLLER, A YOKOTA, M F WHITE, A G PAZIANOS, J S FLIER: "A Naturally Occurring Mutation of Insulin Receptor Alanine 1134 Impairs Tyrosine Kinase Function and Is Associated with Dominantly Inherited Insulin Resistance" JOURNAL OF BIOLOGICAL CHEMISTRY, vol. 265, no. 25, 5 September 1990, pages 14979-14985, XP002088034
- R I BRINKWORTH, D P FAIRLIE: "Non-peptidic anti-AIDS agents: Inhibition of HIV-1 proteinase by disulfonates" BIOCHEMICAL AND BIOPHYSICAL RESEARCH COMMUNICATIONS, vol. 188, no. 2, 30 October 1992, pages 624-630, XP002088035 cited in the application
- W H OJALA, C R OJALA, W B GLEASON: "The X-ray crystal structure of the sulfonated azo dye Congo Red, a non-peptidic inhibitor of HIV-1 protease which also binds to reverse transcriptase and amyloid proteins " ANTIVIRAL CHEMISTRY AND CHEMOTHERAPY, vol. 6, no. 1, 1995, pages 25-33, XP002088036
- G C ROLBAND, J F WILLIAMS, N J G WEBSTER, J M OLEFSKY: "Deletion of the Insulin Receptor beta-Subunit Acidic Domain Results in Enhanced Metabolic Signaling" ENDOCRINOLOGY, vol. 133, no. 3, 1993, pages 1437-1443, XP002088037
- R RAFAELHOFF, B A MADDUX, A BRUNETTI, P SBRACCIA, C K SUNG, R PATEL, D M HAWLEY, I D GOLDFINE: "Transmembrane signalling by insulin via an insulin receptor mutated at tyrosines 1158, 1162 and 1163." BIOCHEMICAL AND BIOPHYSICAL RESEARCH COMMUNICATIONS, vol. 179, no. 2, 16 September 1991, pages 912-918, XP002088038
- B A MADDUX, I D GOLDFINE: "Evidence That Insulin Plus ATP May Induce a Conformational Change in the Beta Subunit of the Insulin Receptor without Inducing Receptor Autophosphorylation" JOURNAL OF BIOLOGICAL CHEMISTRY, vol. 266, no. 11, 15 April 1991, pages 6731-6736, XP002088039
- C K SUNG, X-L HAN, A BRUNETTI, B MADDUX, R YAMAMOTO-HONDA, I D GOLDFINE: "Regulation of Biological Functions by an Insulin Receptor Monoclonal Antibody in Insulin Receptor beta-Subunit Mutants" BIOCHEMISTRY, vol. 31, no. 1, 1992, pages 168-174, XP002088040
- WEI L ET AL: "EXPRESSION, CHARACTERIZATION AND CRYSTALLIZATION OF THE CATALYTIC CORE OF THE HUMAN INSULIN RECEPTOR PROTEIN-TYROSINE KINASE DOMAIN" JOURNAL OF BIOLOGICAL CHEMISTRY, vol. 270, no. 14, 7 April 1995, pages 8122-8130, XP002061073
- HUBBARD S R ET AL: "CRYSTAL STRUCTURE OF THE TYROSINE KINASE DOMAIN OF THE HUMAN INSULIN RECEPTOR" NATURE, vol. 372, 22 December 1994, pages 746-754, XP002061072 cited in the application
- H K KOLE, A S LIOTTA, S KOLE, J ROTH, C MONTROSE-RAFIZADEH, M BERNIER: "A Synthetic Peptide Derived from a COOH-terminal Domain of the Insulin Receptor Specifically Enhances Insulin Receptor Signaling" JOURNAL OF BIOLOGICAL CHEMISTRY, vol. 271, no. 49, 6 December 1996, pages 31619-31626, XP002088041 cited in the application
- R A KOHANSKI: "Insulin Receptor Autophosphorylation. II. Determination of Autophosphorylation Sites by Chemical Sequence Analysis and Identification of the Juxtamembrane Sites" BIOCHEMISTRY, vol. 32, no. 22, 1993, pages 5773-5780, XP002088042 cited in the application
- S R HUBBARD: "Crystal structure of the acfivated insulin receptor tyrosine kinase in complex with peptide substrate and ATP analog" EMBO JOURNAL, vol. 16, no. 18, 1997, pages 5572-5581, XP002088043 cited in the application

## Description

### Technical Field,

The invention relates to modulation of glucose uptake and insulin receptor activity generally. More precisely, the invention is directed to an *in vitro* method to modulate the kinase activity of insulin receptor and/or to potentiate the insulin activation of insulin receptor and/or to potentiate the stimulation by insulin of cellular glucose uptake or to stimulate the uptake of glucose in cells displaying the insulin receptor. In addition, the invention relates to a specific compound and a composition comprising same.

### Background Art

Among the many functions performed by peptides and proteins in metabolism is the ability to stimulate receptors at cell surfaces to effect intracellular consequences important in maintenance and development of the organism. Peptide and protein hormones interact with receptors specific for them so that the activity of the hormone is felt on designated cells exhibiting these receptors. The insulin receptor is present on virtually all cells and at high concentrations on the cells of the liver, skeletal muscles, and adipose tissue. Stimulation of the insulin receptor with insulin is an essential element in carbohydrate metabolism and storage.

Diabetics either lack sufficient endogenous secretion of the insulin hormone (Type 1) or have an insulin receptor-mediated signaling pathway that is to some degree resistant to endogenous or exogenous insulin, either through primary or post-translational structural changes, reduced numbers or poor coupling among signaling components (Type II). All Type I diabetics, and many Type II subjects as well, must utilize injection to obtain enhanced activity of the extant insulin receptors, since endogenous insulin can at present be replaced only with an alternative supply of insulin itself, previously isolated from native sources, and now recombinantly produced. While the recombinant production of insulin permits a less immunogenic form to be provided and assures a reliable supply of needed quantities, the necessity to administer the hormone by injection remains, due to the instability of peptides and proteins in the digestive tract. It has long been the goal to substitute for peptide ligands, including insulin, small molecules which are not digested and can be absorbed directly into the bloodstream. However, to date, nonpeptide substances which can exert the effect of insulin on its receptor have eluded discovery.

There have been many instances in which nonpeptide materials have been used to inhibit enzymes whose native substrates are peptides. For example, Brinkworth, R.I. *et al. Biochem Biophys Res Comm* (1492) 188:624-630 describe the inhibition of HIV-1 proteinase by various aryl disulfonates. The ability of triazine dyes to bind NADH oxidase from *Thermus thermophilus* was studied by Kirchberger, J. *et al. J Chromatog A* (1994) 668:153-164.

It has also been shown that certain nonpeptide components enhance the agonist properties of a peptide hormone. The ability of certain thiazolidinediones such as pioglitazone to enhance adipocyte differentiation by stimulating the effect of insulin has been described by, for example, Kletzien, R.F. *et al. Mol Pharmacol* (1992) 41:393-398. These represent a class of potential antidiabetic compounds that act at an unknown site downstream from the insulin receptor itself and enhance the response of target tissues to insulin. Kobayashi, M. *Diabetes* (1992) 41:476-483. It is now known that most of the thiazolidinediones bind to PPARγ thus triggering certain nuclear events that may result in enhanced sensitivity of the target cells to insulin. However, the complete mechanism is still unresolved.

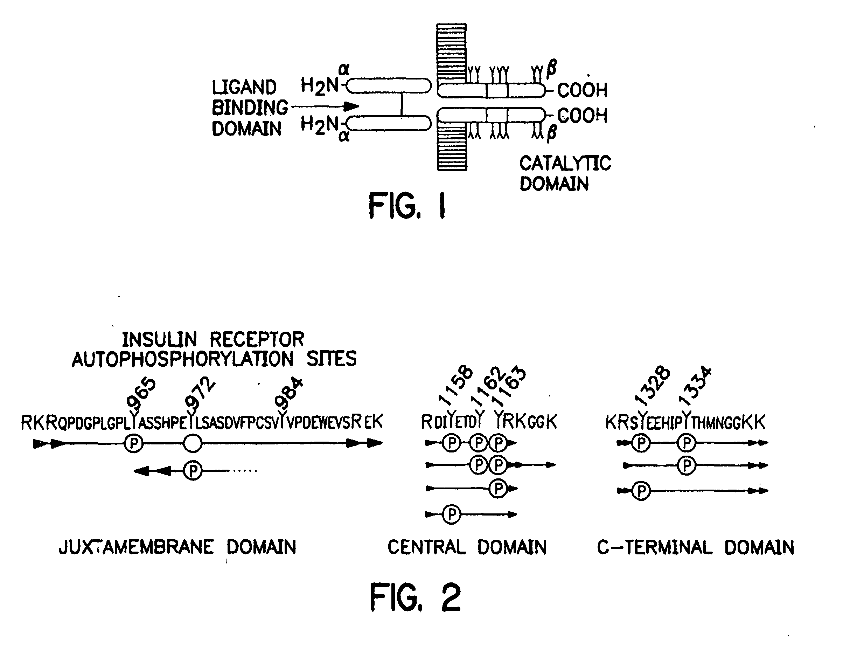

Considerable information is known concerning the insulin receptor itself. The structure of the insulin receptor and some aspects of its mode of action as currently understood, are illustrated in Figure 1. The receptor consists of four separate subunits consisting of two identical α and two identical β chains. The two β chains contain a cross-membrane domain; the α portions are in the extracellular domain and accommodate the binding of insulin. The illustration in Figure 1 shows the site at which insulin binds to the receptor. The β subunits contain a tyrosine kinase activity, shown as the white inserts into the subunits and the kinase of one β subunit effects the phosphorylation of the complementary β subunit as shown; the receptor illustrated in Figure 1 is in its activated form when the tyrosine residues (Y) are phosphorylated. The β subunits also contain ATP binding sites. The insulin-stimulated phosphorylation of the receptor itself is required for subsequent activity and thus demonstration of the ability of a compound to effect phosphorylation of the β subunits provides a means to assay activation of the receptor.

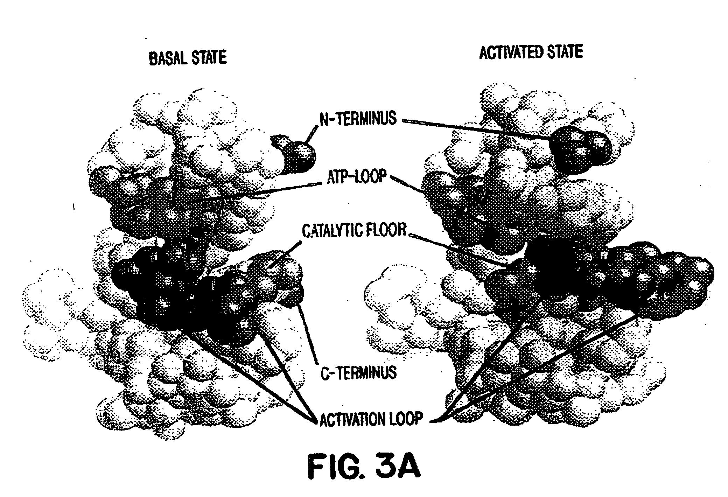

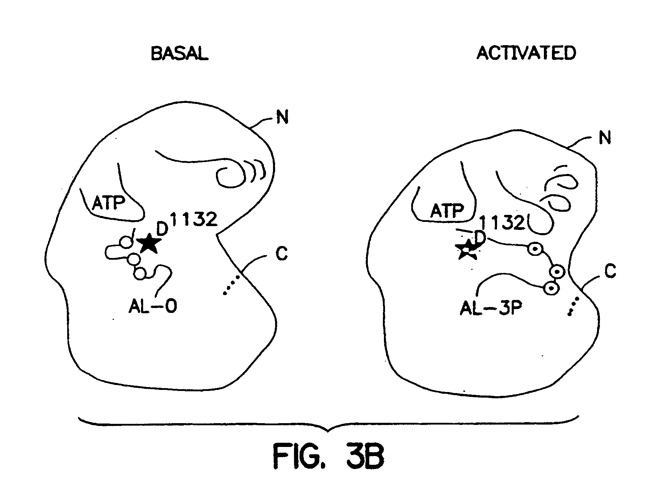

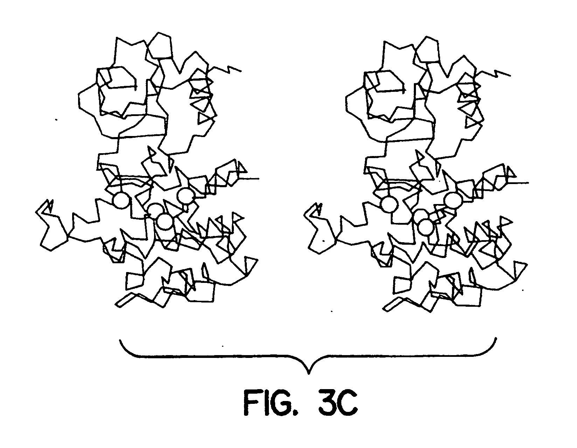

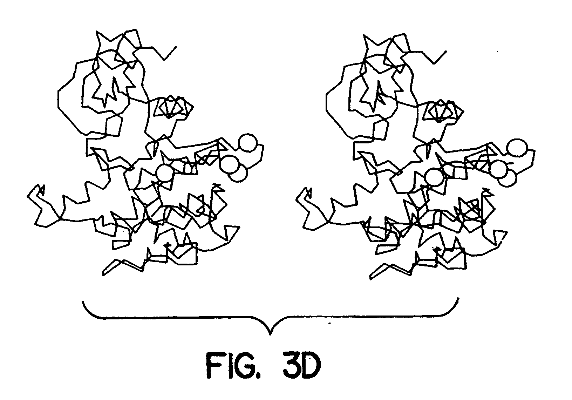

Kohanski, R.A. *Biochemistry* (1993) 32:5773-5780 has described the distribution of the phosphorylated tyrosine residues on the cytoplasmic β chain domains of the insulin receptor (IR). Figure 3 of this article is reprinted as Figure 2 in the present application and shows the several domains which contain phosphorylated tyrosine when the receptor is activated by insulin. These include the juxtamembrane (JM) domain; the central (catalytic) domain (CD); and the C-terminal domain (CT). The location of these regions is also indicated in Figure 1 herein. Thus, the phosphorylated tyrosines are at positions 965, 972, 1158, 1162, 1163, 1328 and 1334 of the human β chain. The central domain appears to be responsible for the autophosphorylation of the tyrosine residues on the complementary chain.

In addition, Hubbard, S.R. *et al. Nature* (1994) 372:746-754 describe the crystal structure of the tyrosine kinase (activation) domain of the receptor. The authors further conclude that tyrosine at position 1162 is present in the active site of the enzyme and effects the inhibition of kinase activity. Upon phosphorylation, tyrosine 1162 no longer occupies the active site.

Kole, H.K. *et al. J Biol Chem* (1996) 271:31619-31626 cite earlier work indicating that kinase regulation by sequences within the carboxy terminus of the insulin receptor β chain may be involved in defining the specificity of downstream signaling events. Antibodies directed against epitopes in the region 1294-1317 (numbering used as by Ulrich, A. *et al. Nature* (1985) 318:756-761) inhibit *in vitro* kinase activity with respect to exogenous substrate. The work reported in the Kole paper shows that a peptide representing amino acid positions 1293-1307 could potentiate the ability of insulin to activate the receptor *in vitro* using partially purified insulin receptors from CHO/HIRc cells with a maximum ranging between 50-100 µM concentration of the peptide. This ability to potentiate insulin activation of the receptor could also be shown *in vivo* using semipermeabilized CHO/HIRc cells. By making the peptide more lipophylic by derivatizing it with a stearyl residue, the same effect could be shown in intact cells. The location of binding of the peptide was shown to be the β chain using semipermeabilized CHO/EI cells, 50 µM biotinylated peptide, and exposure to 0.2 mM BSO-COES, a bifunctional cross-linking reagent, followed by immunoprecipitation with anti-αIR and detection using streptavidin conjugated enzyme. The association of the biotinylated peptide with a 95 kD protein suggested binding to the β subunit. The experiment was repeated using a mutant insulin receptor lacking 43 amino acids from the carboxyl terminus; binding of the peptide was observed at a level higher than in the CHO/EI cells. Thus, the authors conclude that the peptide (1293-1307) binds specifically to the β subunit in a region other than the carboxy terminal 43 amino acids.

Despite the knowledge of the structure of this receptor, including the cited Kole paper, it has not as yet been possible to utilize simple molecules to provide the effect of a peptide hormone by stimulating receptor activity independently of the peptide hormone binding site. However, a number of compounds disclosed herein are able to bind the insulin receptor at a site other than that normally activated by insulin. Several aryl di- or polysulfonate compounds which share certain common structural features are able to effect stimulation of the insulin receptor to activate the autophosphorylation activity required for signal transduction. The availability of these compounds permits construction of assays and comparative procedures for evaluating additional candidate compounds as well as the design and synthesis of therapeutics for primary treatment of insulin resistance and diabetics with the appropriate structural features. It has now been found that various sites on the β chain are included in the binding region for such compounds and that these compounds alter the conformation of the core cytoplasmic kinase domain of the β chain (CKD) which extends into the cytoplasm. This insight opens the door to methods for identifying compounds that have characteristics useful in regulating metabolism generally.

### Disclosure of the Invention

It has been shown that substances that modulate the activity of the IR alter the conformation of the two-lobed form of the CKD. This provides the basis for screening assays for compounds that modulate the receptor. Applicants do not wish to be bound by any specific theory, but it appears that compounds which bind to the "belt" region of the two-lobed CKD, as further described below, essentially alter the interfacial contact between the two lobes of the β receptor chain. Alternatively, the conformational change may be a secondary effect of phosphorylation facilitated by binding of an activating compound. Whatever the dynamic sequence, because there are convenient ways to measure changes in conformation, this change in three-dimensional structure, which is closely associated with activation, is suitable for assessing the ability of a test substance to modulate receptor activity. In certain forms, such assays can be adapted for high throughput screening.

In addition, or in the alternative, the invention relates to assays that are based on identification of the binding sites in the β chains for small molecules that modulate the receptor, either directly or in conjunction with insulin stimulation or both. A small molecule. TER16998. described below, is able to activate the insulin receptor through interaction with the β chains alone. This activation is inhibited by specific peptides that represent portions of the β chain. Presumably, these peptides act as a "sponge" to interact with TER16998, thus preventing its interaction with the receptor β chains *per se*. Mapping of these peptides to the sites they occupy on the β chains of the receptor identifies regions of binding. In addition, the activation of the receptor by TER16998 provides a result wherein the tyrosines ordinarily phosphorylated upon insulin activation in the JM domain do not become phosphorylated. and wherein phosphorylation of these residues is prevented even in the presence of insulin when TER 16998 is present. The remaining five tyrosine residues that are phosphorylated in the activated receptor and that mediate the activity of the receptor are phosphorylated in the presence of insulin, TER16998, and in the presence of both insulin and TER16998. This result may show that the JM domain is an effective activation target. The regions that are identified as binding sites may be effectively used for identifying substances which will modulate the activity of this receptor.

In one aspect, the invention is directed to *in vitro* methods to modulate the kinase activity of insulin receptor and/or to potentiate the insulin activation of insulin receptor and/or to potentiate the stimulation by insulin of cellular glucose uptake and/or to stimulate the uptake of glucose in cells displaying the insulin receptor which method comprises contacting said insulin receptor or the kinase portion thereof or said cells with a compound which comprises the Formula 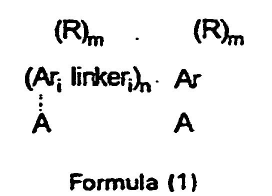 wherein each Ar is independently an aromatic moiety selected from benzene, naphthalene, pyridine, quinoline or benzothiazole;
each A is independently -SO₃X, OP(OX)₃,-COOX, where X is a hydrogen atom or a cation;
each R is independently a substituted or unsubstituted hydrocarbyl moiety, branched or unbranched, cyclic, aromatic or nonaromatic, wherein unbranched hydrocarbyl chains may be interrupted by one or more heteroatoms ; or each R is independently OR', NR'₂ or SR', wherein R' is H or R as defined above;
m is 0, 1 or 2;
n is 1-6; and
each linker is independently -CH₂-, -N=N- -CH=CH-, -NHCO- or -NHCONH-
or an isostere thereof, wherein when n is 1, at least one Ar must comprise at least 2 fused aromatic rings.

In these methods, the cells may be contained in a subject and contacted with the compounds of Formula (1) by administering these compounds to the subject.

In particular embodiments, the compound of Formula (1) has the Formula 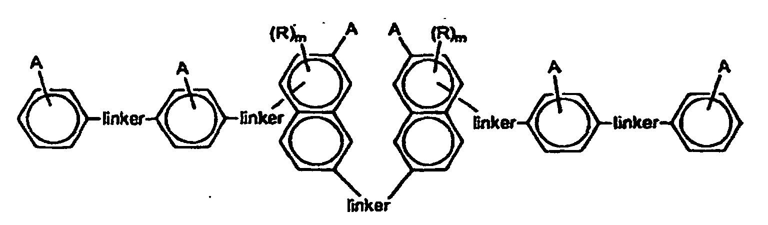 wherein each R, A, linker, and m are as defined above, or of the formula 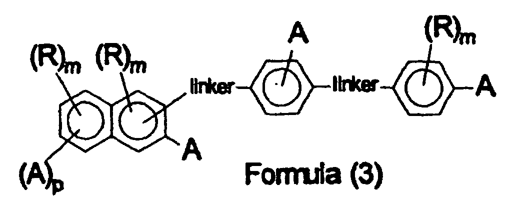 wherein each of R, A, linker and m are as defined above and wherein p is 0 or 1.

In other aspects, the invention is directed to methods to design and synthesize molecules that activate the insulin receptor which are based on the relevant characterizing properties of the compounds of Formula (1).

### Brief Descriptions of the Drawings

Figure 1 shows a schematic diagram of the insulin receptor and its activation by insulin.
Figure 2 shows the locations of the tyrosine residues on the IR β chain which are phosphorylated upon activation of the IR by insulin.
Figures 3A to 3D show the conformational change in the double-lobed CKD region of the insulin receptor when it is activated as compared to its inactive state. Figure 3A shows a space-filling model of the basal state and the activated state of the CKD. The N-terminus, the ATP loop, the activation loop and the catalytic floor are labeled. Figure 3B shows a simplified schematic of the CKD shown in Figure 3A. When the receptor is activated, the activation loop moves dramatically as the tyrosines become phosphorylated; the ATP binding site expands; the helix rotates down as the upper lobe rotates outward from the plane of the paper and the catalytic site containing aspartate, labeled D in Figure 3B, is exposed.
Figures 3C and 3D show stereo views of the basal state and activated state, respectively. Tyrosines 1158, 1162, and 1163 are shown as white spheres and aspartic 1132 by a dark sphere.
Figure 4A shows the structure of TER16998; Figure 4B shows the ability of TER16998 to stimulate autophosphorylation of the IR; Figure 4C shows the ability of TER 16998 to potentiate insulin-stimulated glucose uptake in 3T3-L1 cells.
Figure 5 shows the ability of TER16998 to stimulate the ATPase activity of the two-lobed CKD region of the human insulin receptor.
Figure 6 shows the HPLC pattern of a tryptic digest of the phosphorylated β chain of IR. Trace A shows the digest of the full-length CKD; trace B represents the digest of a truncated version missing 10 kD at the C-terminus.
Figure 7 shows the synthetic pathway for photoaffinity labeled TER 16998.
Figures 8A-8E show the structures of several compounds relevant to the invention which activate the insulin receptor. Figure 8A shows the structure of TER3935. Figure 8B shows the structures of TER17004 and 17005. Figure 8C shows the structure of TER17003. Figure 8D shows the structure of TER12, Cibacron Brilliant Red 3BA. Figure 8E shows the structure of TER3938, Direct Yellow 27.
Figure 9 shows a pathway for synthesis of TER12.
Figure 10 shows the effect of Component A on insulin-induced uptake of glucose by adipocytes.
Figure 11 shows the synthetic pathway for TER 16998.
Figure 12 shows the effect of TER16998, alone and in combination with insulin, on autophosphorylation of the IR receptor.
Figure 13 shows the effect of TER16998 on insulin-induced glucose uptake in adipocytes.
Figure 14 shows the effect of TER16998 on blood glucose levels in a diabetic mouse model.

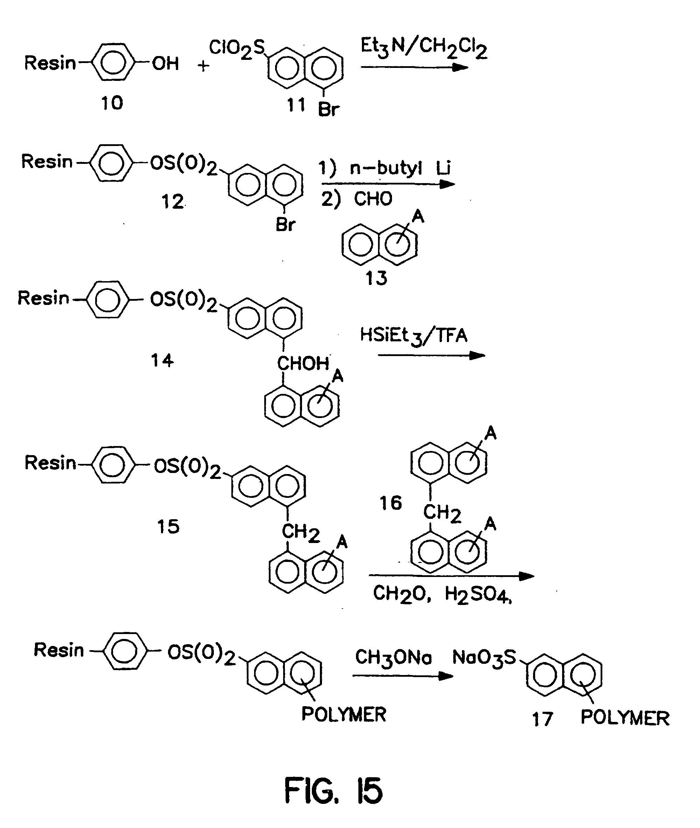

Figure 15 shows the synthetic method for preparation of analogs of TER3935 with alternative linkages.
Figure 16 shows the synthesis of TER17003.
Figure 17 shows the synthesis of TER17004 and TER17005.
Figures 18A and 18B show the location on the CKD of the human insulin receptor of peptides which inhibit the activation of TER16998.
Figure 19 shows the results of an experiment which demonstrate that TER16998 is taken up by cells and is consistent with the ability of TER16998 to enhance the stimulation of glucose uptake by insulin through its direct interaction with the CKD.
Figure 20 shows the insulin sensitizing effect of TER16998 in diabetic mice.

### Modes of Carrying Out the Invention

The invention is directed to methods to regulate and manage subjects with diabetes by virtue of administering compounds which are found to modulate the IR by virtue of the assays of the invention. These compounds act directly on the core kinase domain of the receptor and do not compete with insulin for binding at the insulin-binding site, nor do they effect activation of the receptor by a mechanism similar to that exhibited by insulin. The compounds of the invention are able directly to activate the kinase of the receptor to autophosphorylate, thus to potentiate the effect of insulin on the receptor, to activate the kinase function of the receptor in phosphorylating exogenous substrates, to effect the increased uptake of glucose by adipocytes and insulin receptor-bearing cells in general, and to lower blood glucose levels in diabetic subjects.

The invention is concerned with modulation (activation) of the insulin receptor. As set forth hereinabove, the overall physiological aspects resulting from activation of the receptor are known: the activated receptor has kinase activity, insulin activation stimulates cellular glucose uptake and if the cell is in a mammalian subject, lowers blood glucose. The downstream events associated with activation are also known. These include those set forth above plus phosphorylation of IRS-1, stimulating the kinase activity relative to phosphoinositol (PI₃ kinase activity) and ultimately effecting GLUT-4 glucose transporter translocation to the cell surface. Thus, as described below, the compounds identified by the method of the invention and the specific compounds described below act directly on the insulin receptor CKD and cause these downstream events.

The crystal structure of the insulin receptor has been determined, and the general conformation of the receptor is shown in Figures 3A-3D. Details of the crystal structure of the basal form are set forth in Hubbard, S.R. *et al*. (1994, *supra,* incorporated herein by reference) and for the activated insulin receptor tyrosine kinase in a complex with peptide substrate and an ATP analog are set forth in Hubbard, S.R. *EMBO J* (1997) 16:5572-5581. The three-dimensional structure of the receptor may be studied using Biosym software to model changes in conformation in the activated state.

The conformational changes which occur when the receptor is activated are apparent from these figures. As shown, upon activation, the activation loop (AL in Figure 3B) moves its position dramatically as the tyrosines are phosphorylated, as indicated in the diagram. In addition, the ATP binding site expands and the helix residues 1038-1051 shown at the upper right rotates down as the upper N-terminal lobe rotates relative to the lower C-terminal lobe. Finally, the aspartate at position 1132 in the catalytic domain is exposed.

### Screening Assays for IR Modulators

The invention takes advantage of the conformational change which occurs in the two-lobed CKD region of the insulin receptor upon activation. In order to make effective utilization of this property, an assay must be adapted which permits detection of this change without the time-consuming requirements of, for example, direct detection by x-ray crystallography. The conformational change can, indeed, be detected in a high throughput assay by assessing secondary measures of its occurrence. These include employing the two-lobed CKD in fluorescence-labeled form; the lobes are labeled with donor and recipient fluorophores and the distance between donor and receptor can be measured by standard fluorescence energy transfer determination techniques. Any one of a multiplicity of fluorophores is available commercially for practice of this technique, and determination of distance by fluorescence energy transfer is a well known laboratory procedure.

Other, more indirect measures of conformational change can also be employed. It should be noted that the conformational change, always associated with activation, may not be the initial response to an activator, but may be the consequence of other, initiating, changes in the receptor. For example, an activating compound may dimerize two CKDs resulting in a higher local density of catalytic site and activation loop, which would then lead to initial phosphorylation which in turn would lead to the conformational change. The conformational change would result in a measurable kinase activity that can be assayed on exogenous substrates. Alternatively, the activating compound may wedge into the CKD so that the activation loop becomes exposed to solvent and thus subject to phosphorylation, without inducing the rotation of the two lobes relative to each other until later. In any event, it is clear that during the activation of the receptor, conformational change does occur and this can be measured as described above or, more indirectly, as described below.

For example, the ATPase activity of the two-lobed CKD can be measured. This has been illustrated using TER16998 as a typical activator of the receptor which interacts directly with the CKD. Indeed, the ability of this activator to result in ATPase activity confirms the existence of conformational change since the basal IR exhibits only low ATPase activity. Thus, the catalytic site has been exposed as a result of activation.

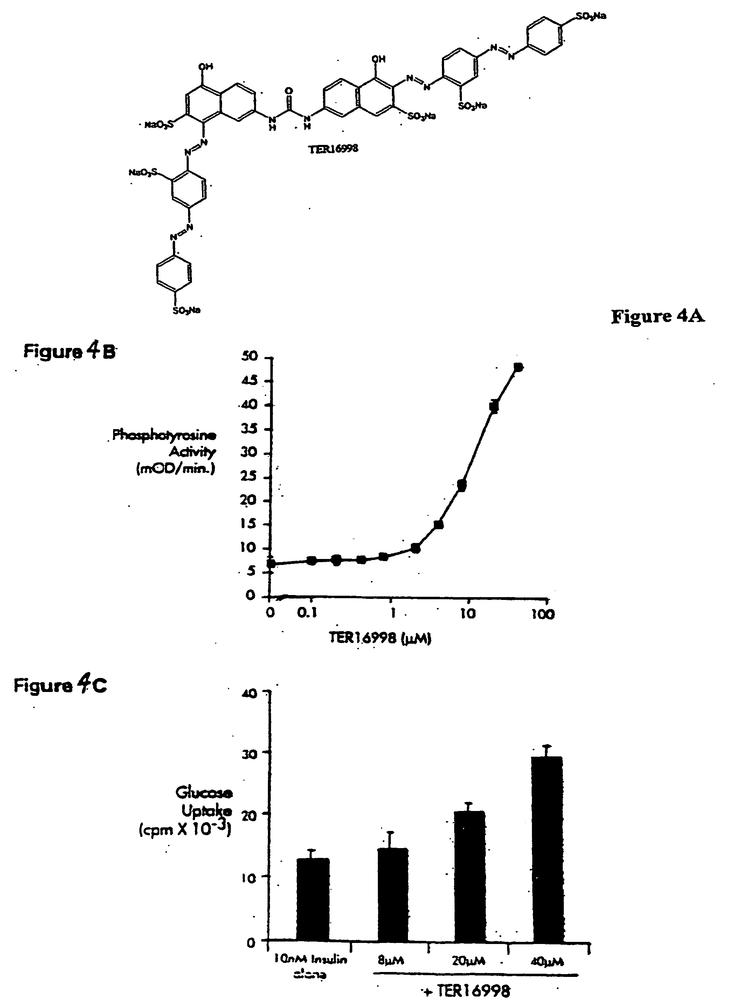

TER16998 has been confirmed as an activator of the receptor, as illustrated in Figure 4. Figure 4A shows the structure of TER16998. Figure 4B shows the ability of TER16998 to activate the solubilized receptor directly *in vitro* as shown by enhanced phosphorylation of the tyrosine residues. Figure 4C illustrates the ability of TER16998 to enhance the effect of insulin on the receptor to stimulate the glucose uptake by cells. Increasing amounts of TER16998 in the presence of insulin show a dose response curve consistent with this stimulation.

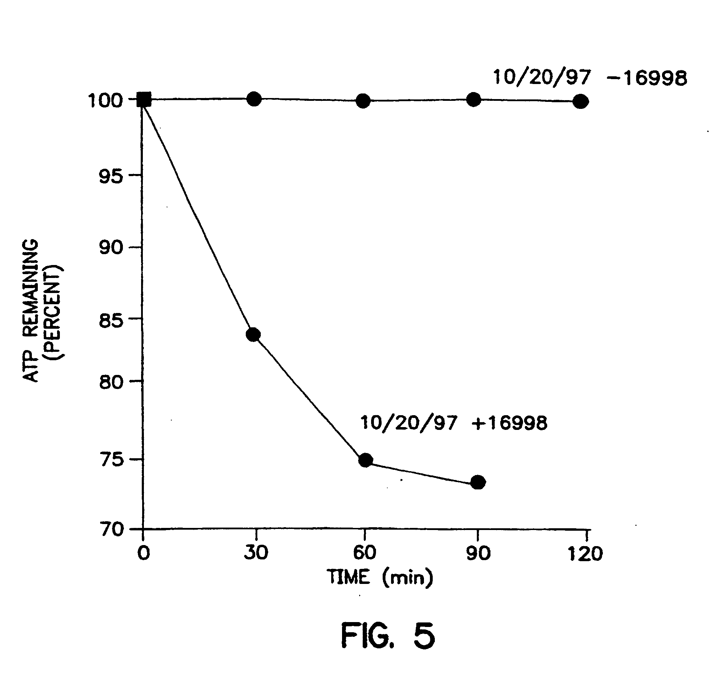

The ability of the established activator, TER16998, then, is confirmed to effect the conformational change that results in exposure of the ATPase catalytic site as shown in Figure 5. The human insulin receptor CKD exhibits only low ATPase activity in the absence of TER16998, but has this activity in its presence. The results in Figure 5 were obtained using an HPLC method for evaluating the effect of TER16998 on ATPase activity. The reaction mixture contains 25 mM Tris pH 7.4, 2 mM MnCl₂, 8 mM MgCl₂, 0.4 µg/ml human IR-CKD, and 5 mM ATP at 30°C. The reaction is conducted in the presence and the absence of 2 µg/ml TER16998. The reaction mixture is placed over a Rainin Microsorb-MV Column (4.6 x 150 mm) in a buffer containing 120 mM ammonium phosphate and 11.9 mM tetrabutyl ammonium hydrogen sulfate, pH 6.5, in methanol. The rate of hydrolysis was calculated using the peak areas ADP/(ADP+ATP) and ATP/(ADP+ATP) and then plotted as percent ATP remaining versus time.

Alternatively, the conformational change can be measured indirectly by measuring the state of phosphorylation of the double-lobed CKD. One embodiment of this assay is a modified form of that described in Hagino, H. *et al. Diabetes* (1994) 43:274-280. Briefly, human insulin receptors (hIR) are partially purified from placental extracts or from cell line IM-9. The partially purified hIRs are captured into microplate wells by incubating them for 90 minutes with wells coated with a monoclonal antibody to hIR. The wells are then treated with various dose levels of insulin and/or test compounds for 15 minutes at room temperature; ATP (10 µM) was then added to permit kinase activity to proceed. After 60 minutes, the wells are washed, and then treated for 60 minutes with biotinylated antibody directed against phosphotyrosine (PY-20) and unbound materials again washed away. The wells are then incubated with a conventional streptavidin peroxidase system for 30 minutes to assess the level of phosphorylated tyrosine. When tested in this assay, insulin gave a dose response curve showing an EC₅₀ of about 0.3 nM and a maximal activity at about 100 nM. The EC₅₀ is similar to that obtained for binding of labeled insulin to various cells and tissues.

The kinase activity of a double-lobed CKD can also be measured on exogenous substrates, as a secondary consequence of the conformational change. In one embodiment, poly(Glu₄Tyr) can be used as the substrate for phosphorylation. Incorporation of labeled phosphate from γ-labeled ATP is measured following activation of receptor prepared as above or by substituting, for the human insulin receptor, a recombinantly produced β chain lacking the insulin-binding domain (supplied by Stratagene, Inc.). These assays can be run in nonradioactive form at high throughput by means of a fluorescence polarization displacement assay, for example.

The conformational change in the two-lobed CKD region is closely associated with the activation of the receptor, regardless of whether it is the initial, or itself a secondary, result of the association of an activator molecule.

The methods set forth above, when used generally to screen for modulators of the IR, can be optimized by using a set of maximally diverse candidate compounds in the initial screens. This method comprises, in a preferred embodiment, contacting each member of a set of maximally diverse candidate compounds with said receptor or in particular, the double-lobed CKD portion thereof; detecting a conformational change, or kinase activity or phosphorylation of tyrosine on the CKD contacted with each set member, and identifying as a successful candidate at least one member of the set wherein a conformational change, or kinase activity or an increased amount of tyrosine phosphate relative to untreated receptor is detected in the CKD with which it was contacted.

In general, once a compound with at least moderate ability to activate the insulin receptor has been identified, additional compounds can be identified by comparing the properties of the candidates with those of compounds having known activity. One particularly useful property to compare is the affinity fingerprint of the compound against a reference panel of proteins which provide a first approximation of the binding modes of all proteins, as described in U.S. Patent No. 5.587.293.

Further, analysis of compounds shown to activate the insulin receptor using standard structure activity analysis will result in additional compounds which behave as activators. Receptor mutagenesis and photoaffinity analogs, as described below, may also be used to identify the receptor site binding the compounds, for use in rational drug design.

The activator compounds are able to alter the conformation of the two-lobed CKD and/or stimulate the phosphorylation catalyzed by IR kinase alone, i.e., to behave as agonists with respect to the receptor and/or are able to enhance the ability of insulin to effect phosphorylation of the receptor. Any of these effects can be considered an activation of the insulin receptor. Thus, by "activating" the insulin receptor is meant either the ability to behave as an agonist or the ability to enhance the stimulation by insulin or other agonists of the receptor activity. Both of these effects can be evidenced by conformational change and/or autophosphorylation of the receptor and/or kinase activity thereof.

### Identification of Critical Sites

The invention also is directed to the identification of the apparent active sites in the cytoplasmic domain affected by interaction with a modulator/activator of the receptor. Identification of such sites is useful in order to design assays to identify compounds that will have a variety of metabolic effects, all related to modulating the activation of the insulin receptor. At least two types of assays can be devised based on identification of a site involved directly in activation. First, the amino acid sequences associated with this site can be used directly in assays to measure formation of complexes with potential modulators of the receptor. One way in which modulators can activate the receptor is by directly binding these sites. A second type of assay involves modifying sites that are critical for activation and comparing the effect of candidate compounds on the native receptor as compared to the modified form. The interaction measured may be simply binding, or other measure of receptor response, such as kinase activity.

As further described hereinbelow, several sites on the β chain of the receptor have been identified as directly involved in activation of the receptor. These include three domains deduced from experiments whereby peptides representing these regions on the CKD have been shown to be associated with binding of the modulator TER16998 by virtue of their ability to inhibit the activation of the CKD by this compound. These domains comprise positions 1099-1105, 1120-1137, and 1235-1252. Another domain which is involved in activation is the juxtamembrane (JM) domain which may directly bind the activating compound, or may be removed from the catalytic site by the conformational change that occurs upon activation elsewhere.

As described below, TER16998 or an alternative compound known to activate the β chains directly can be used *per se* in localizing the binding site relevant to activation or a derivative of this compound can be used. By "derivative" is meant a chemically modified form of the activating compound which retains the binding activities vis-à-vis the insulin receptor exhibited by the parent compound. Particularly important derivatives are those which provide a means for covalent binding through photoactivation. Other derivatives include labeled forms which may aid in detection of the binding site, and minor modifications in structure which do not interfere with the interaction of the compound with the binding site of the parent compound in the IR.

Once the binding site is identified, compounds that activate the receptor can be identified by direct determination of their ability to complex with these sites; or by determining, from the three-dimensional structure of the receptor, the critical residues that participate in activation of the receptor through binding of this type, followed by mutations of these residues so that a differential effect of binding on mutants versus native receptor can be determined; and by complementarity to the three-dimensional structure of the binding region.

In determining the ability of a candidate substance to interact with the identified site, actual physical experimentation is typically employed. However, in the alternative, virtual interaction can also be assessed. Thus, "contacting" the identified site of the receptor with a test composition includes not only physical contact, but also virtual contact -- i.e., docking of a derived pharmacophore to residues identified in the crystal structure or in modeled extension thereof. Thus, as described below, the relevant features of the target site in the appropriate regions can be determined through evaluation of the crystal structure and the particulars of the binding of a model compound, such as TER16998 to this domain. Accordingly, the three-dimensional target site can be modeled and the features of the pharmacophore deduced based on complementarity to the target. The phannacophore will be defined in terms of parameters such as electrostatic potential distribution -- i.e., the general shape of the electron cloud pattern. The pharmacophore thus described can also be used to provide a model for salient features of the structures of known molecules. This information can be combined with the fingerprints determined as described below for these molecules used as comparative standards to select additional compounds with similar characteristics.

It has been determined that relevant portions of the IR β chain that participate in binding modulating compounds are represented by positions 1089-1105, by positions 1120-1137 and by positions 1235-1252, and by inference, residues spatially adjacent thereto. (See Example 7.) These critical domains were identified by constructing a series of 14 peptides representing various domains of the CKD of the human form of the insulin receptor and testing the ability of these peptides to inhibit the activation of the CKD kinase activity by TER16998. In general, peptides which are able to bind the activating compound will inhibit this activity because of their behavior as decoys or sponges for the activator. Thus, in general, positions which bind activators can be identified by constructing peptides corresponding to the relevant domains on the β chain and testing these peptides for their ability to act as sponges in any *in vitro* assay where the ability of a known activator to effect the conformational change in the two-lobed CKD is determined by any suitable assay as described above. Peptides which contain a site which participates in the binding of the activator will inhibit the activation. In addition to the sites contained in the peptides themselves. residues that are spatially proximal to the binding site identified in the inhibiting peptide in the three-dimensional structure of the CKD also participate in binding the activator.

In addition to the domains set forth above, the JM domain has also been implicated in activation caused by compounds like TER16998 by virtue of the demonstration that the tyrosines contained in the JM domain are not phosphorylated when the receptor is activated by TER16998.

The JM domain is defined in accordance with the illustration in Figures 1 and 2. As shown in Figure 1, the JM domain is contained in the cytoplasm immediately adjacent to the transmembrane region of the IR β chains. The extent of this region is as defined by Kohanski, using the numbering of Ebina, Y. *et al. Cell* (1985) 40:747. The JM domain contains two tyrosines that are autophosphorylated when the receptor is activated by insulin, at positions 965 and 972. The tyrosine at position 984 is apparently not phosphorylated.

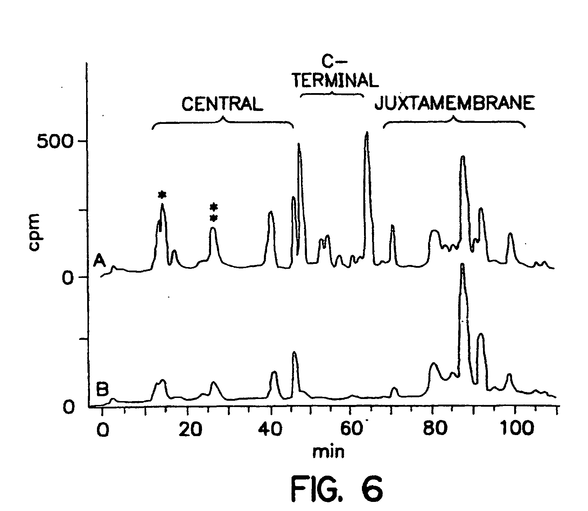

The phosphorylation status of the various regions of the cytoplasmic domain under various conditions is determined by the method of Kohanski (1993, *supra*, using tryptic digestion of the phosphorylated cytoplasmic region. As shown by Kohanski, and illustrated in Figure 6 herein (which is Figure 4 of the Kohanski paper), separation of the tryptic fragments on high-resolution HPLC is used to identify the tyrosine residues which have been phosphorylated. Trace A shows the digest of the full-length CKD; trace B represents the digest of a truncated version missing 10 kD at the C-terminus.

Briefly, in Kohanski's work, the cytoplasmic domain is phosphorylated in the presence of γ-labeled ATP. The conditions include 1-10 mM Mn⁺², 10 µM total ATP, 10 µg/ml CKD, with incubation for 2 hrs at 25°C. In testing TER16998 or insulin or both, solubilized intact receptor, containing both α and β chains is used. The phosphorylated β chain containing the cytoplasmic kinase domain (CKD) labeled with radioactive phosphate is then recovered and purified using SDS PAGE. After locating the labeled CKD by autoradiography from the wet, unfixed gel immediately after electrophoresis, the relevant segments are excised from the gel and soaked in water for 30 min. The segments are crushed and treated with trypsin at 0.1 mg/ml as added in 50 mM N-ethylmorpholine acetic acid (pH 7) for 15 hours at room temperature. The tryptic fragments are then recovered by filtration and subjected to HPLC in two steps. The digests are subjected first to anionic exchange on SynChropak AX300 weak anion exchange and gradient-eluted; fractions are pooled according to the distribution of radioactivity.

Each pool is then subjected to HPLC on a Spherisorb C8 reverse phase column and after gradient elution individual fractions recovered. The fractions are identified by comparison to comparable HPLC run on the original tryptic digest. Individual fractions are then sequenced to determine the location of the phosphorylated tyrosines.

This procedure was employed on phosphorylated CKD from intact receptor that had been treated with and without TER16998 and/or insulin.

Table 1 shows the results of this determination:

**Table 1**

| | **Juxtamembrane** | | **Central Domain** | | | **C-terminal** | |
|---|---|---|---|---|---|---|---|
| pY Sites | 965 | 972 | 1158 | 1162 | 1163 | 1328 | 1334 |
| Resting State | X | X | X | X | X | X | X |
| with insulin | P | P | P | P | P | P | P |
| with TER16998 | X | X | P | P | P | P | P |
| with TER16998 and insulin | X | X | P | P | P | P | P |

In Table 1, "X" indicates that the relevant tyrosine is not phosphorylated; "P" indicates that it is.

It is apparent from the results in Table 1 that insulin activation of the receptor results in phosphorylation in all regions; activation by TER16998 may effect phosphorylations in the central domain and the C-terminal domain by virtue of its own interaction with the JM domain since when both insulin and TER16998 are used in combination, the JM tyrosine sites remain unphosphorylated while the remaining sites, characteristic of the activated receptor, are converted to phosphotyrosine.

The composition or substance which can agonize the insulin receptor by modulating the JM region does not necessarily need to mimic the electron cloud pattern of phosphorylated residues in this region since it appears that the JM tyrosines do not need to pick up a phosphate in order to activate the receptor. This is known because mutating the JM tyrosines does not result in the inability of insulin to activate the receptor while mutating the tyrosines in the catalytic domain destroys insulin activation of the receptor. The JM tyrosines are apparently phosphorylated in *cis* kinase reaction which in turn facilitates trans kinase reaction on the remaining tyrosine residues.

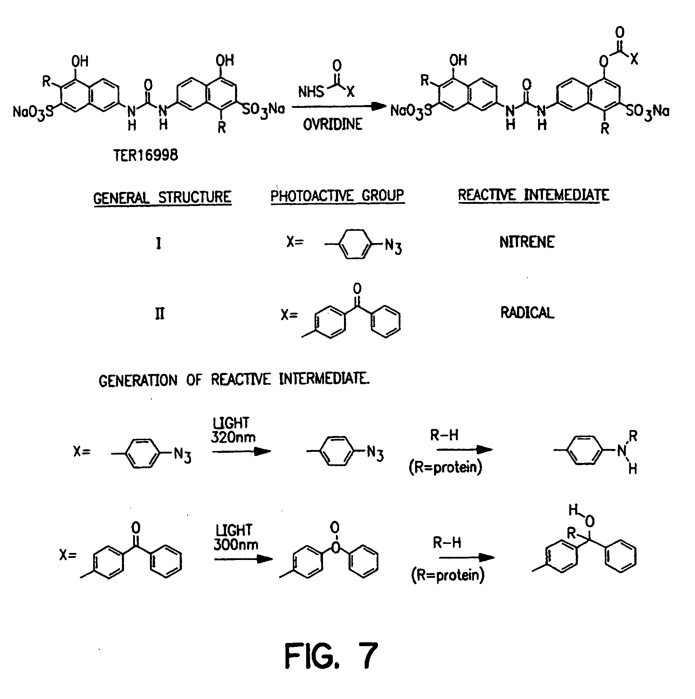

As set forth above, the binding location of TER16998 or other activator can be further defined by converting the noncovalent association of the activator to the CKD region to a covalent one through, for example, the use of a photoaffinity label, followed by locating the covalently bound labels by sequencing. Thus, when the activator is associated with the target region, light activation results in covalent binding. Figure 7 shows an illustrative synthesis of photoaffmity probes for the binding of TER16998. Both nitrene and radical-forming embodiments are shown, although photoactivity of the azo linkages in the TER16998 itself could also be used. As shown, a naphthyl hydroxy substituent is esterified using N-hydroxysuccinimide (NHS) to result in attachment of the photoactivating moiety. Upon activation with light of suitable wavelength, the TER16998 derivative will then become covalently bound to its associated position on the receptor as shown. This permits more accurate definition of the point at which TER16998 is associated. In the alternative, TER 16998 can be used *per se* followed by treating with bifunctional linkers such as those available from Pierce Chemical Company, Rockford, IL, so as to effect covalent attachment of TER16998 to the appropriate site.

The availability of the three-dimensional structure of the receptor permits identification of residues that are important contributors to the binding of activators in the CKD region. Mutations of these residues can easily be provided, due to the availability of the gene encoding the receptor. Dillalba, M. *et al. Proc Natl Acad Sci USA* (1989) 86:7848-7852. Site-directed mutagenesis will thus provide modified forms of the protein wherein successful binding to a test compound is irrelevant to its ability to show IR activation and loss of binding to a compound by the mutant IR is relevant to IR activation. Thus, these mutants can be used as differential screening substrates in combination with the native protein or irrelevant mutants to distinguish between compounds that simply bind the receptor and those that are capable of activating it by binding to the CKD region.

Among substances which activate the insulin receptor will be included antibodies or fragments thereof which are immunospecific for the identified activator binding sites further described below, since such antibodies will specifically bind this region, shown to be involved in activation of the receptor. Preparation of such antibodies is readily accomplished by well established methods, typically involving immunization of a mammal or other vertebrate with the peptide representing the site optionally coupled with an immunogenic carrier. Antibodies may be obtained directly from the plasma of the immunized animal or, preferably, monoclonal antibodies may be obtained using conventional hybridoma technology. Such hybridomas are also useful sources for the genes encoding the relevant monoclonal antibodies which can then be manipulated to obtain these or modified antibodies using recombinant techniques. This makes possible the preparation not only of antigen binding fragments of the antibody (which could be obtained directly from the polyclonal antisera or the monoclonal antibodies secreted by hybridomas through enzymatic cleavage) but also permits single-chain forms, for example, Fᵥ forms to be produced. Alternatively, random libraries of recombinant Fᵥ forms can be screened. The availability of the genes also allows for modifications of the antibodies to investigate the criticality of the variable region to elucidate the nuances of the interaction with receptor. Such antibodies thus represent not only compounds which may directly activate the receptor, but also useful tools for the design of small molecules which bind the identified sites.

### Compounds that Activate the Insulin Receptor

A number of compounds that are able to activate the insulin receptor through direct interaction with the CKD have been identified. This class of compounds has the general Formula 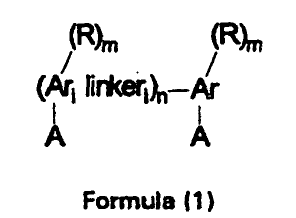 wherein each Ar is independently an aromatic moiety;
each A is independently a photon-accepting substituent;
each R is independently a noninterfering substituent;
m is 0, 1 or 2;
n is 1-6; and
each linker is independently -CH₂-, -N=N- -CH=CH-, -NHCO- or -NHCONH- or an isostere thereof, wherein when n is 1, at least one Ar must comprise at least 2 fused aromatic rings.

A specific example of the compound of Formula (1) is represented by component A of the formula:

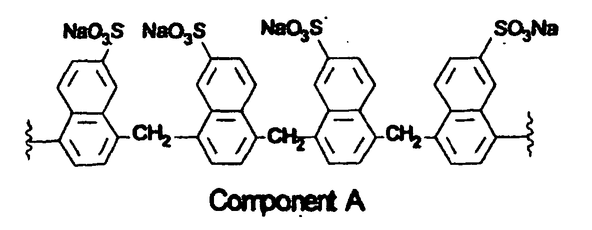

In the compounds of Formula (1), the proton-accepting substituents represented by "A" may be anionic or may be sufficiently nucleophilic to accept a proton at physiological pH. Particularly preferred embodiments of A include -SO₃X, OP(OX)₃ and -COOX where X is a hydrogen atom or a cation depending on pH. Suitable cations include inorganic cations such as sodium, potassium, calcium and the like or may be organic cations such as those provided by organic bases, for example, caffeine. Also included in embodiments of A are amino substituents including primary, secondary, and tertiary amines. Typical bioisosteres of anionic ligands such as tetrazole rings, even when they are not charged.

The aromatic moieties represented by Ar are monocyclic or bicyclic aromatic systems such as benzene or naphthalene or contain one or more heteroatoms selected from the group consisting of O, S and N. Thus, included among the aromatic systems are benzothiazoles, quinolines, pyridine, and the like. Particularly preferred are naphthylene residues.

The noninterfering substituents on the naphthyl moieties in Formula (1) may or may not be present -- i.e., each m is independently 0 or 1. The position of R is arbitrary in each ease; preferred embodiments of R include substituted or unsubstituted hydrocarbyl moieties, whether straight-chain, branched or cyclic and whether aromatic or nonaromatic. Among these are included but not limited to alkyl substituents of 1-6C, alkenyl substituents of 1-6C, and alkyl or alkenyl substituents wherein the carbon chain is interrupted by one or more heteroatoms such as O, N or S. Substituents may also be of the formula -OR', -NR'₂ and -SR', wherein R' is H or is R as defined above. Particularly preferred embodiments include alkyl (1-6C).

In the compounds of Formula (1), each linker is independently an isostere of -CH=CH- such as -N=N-, -CH=N- or -CH₂CH₂- or -NHCH₂-, or -CH₂- such as NH or O, or of -NHCO- such as -OCO- or -COO-. General methods for forming all of these linkages between aromatic systems are well known in the art.

Particularly preferred compounds of Formula (1) are those wherein each R is alkyl (1-6C). Also preferred are compounds of Formula (1) wherein each m is 0.

Also preferred are embodiments wherein the compound of Formula (1) is 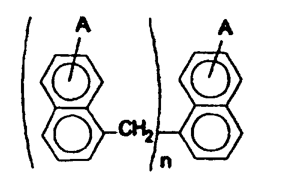 wherein n is 4-6 and A is as defined in claim 1.

Particularly preferred is the compound shown as Component A.

Another preferred form of the compounds of Formula (1) is represented by Formula (2): 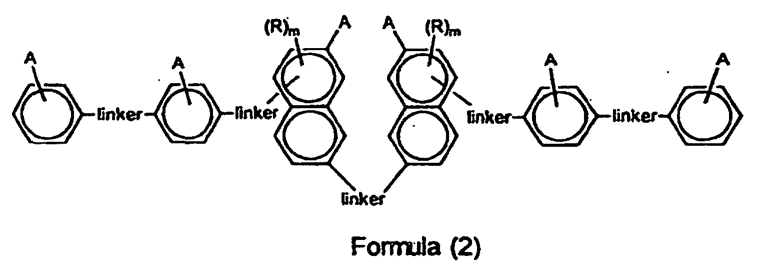 wherein each R, A, linker, and m are as defined above.

Preferred are compounds wherein
each n is independently 0. 1, or 2; and
each linker is independently an isostere of -NHCONH- or of -N=N- or of -NHCO-.

In the compounds of Formula (2) the proton-accepting substituents represented by "A" may be anionic or may be sufficiently nucicophilic to accept a proton at physiological pH. Particularly preferred embodiments of A include -SO₃X, OP(OX)₃ and -COOX where X is a hydrogen atom or a cation depending on pH. Suitable cations include inorganic cations such as sodium, potassium, calcium and the like or may be organic cations such as those provided by organic bases, for example, caffeine. Also included in embodiments of A are amino substituents including primary, secondary, and tertiary amines, as well as typical bioisosteres of anionic ligands such as tetrazole rings, even when they are not charged.

The noninterfering substituents on the naphthyl moieties in Formula (2) may or may not be present -- i.e., each n is independently 0, 1 or 2. The position of R is arbitrary in each case; preferred embodiments of R include substituted or unsubstituted hydrocarbyl moieties, whether straight-chain, branched or cyclic and whether aromatic or nonaromatic. Among these are included but not limited to alkyl substituents of 1-6C, alkenyl substituents of 1-6C, and alkyl or alkenyt substituents wherein the carbon chain is interrupted by one or more heteroatoms such as O, N or S. Substituents may also be of the formula -OR', -NR'₂ and -SR', wherein R' is H or is R as defined above. Particularly preferred embodiments include -OH and additional aromatic moieties containing proton-accepting substituents.

In the compounds of Formula (2), each linker is independently an isostere of -NHCONH-, such as -NHCNHNH-, -NHCOO- or -OCOO- or may be an isostere of -N=N-, such as -CH=CH-, -CH=N- or -CH₂CH₂- or -NHCH₂-. The linkers may also be isosteres of -NHCO-, such as -OCO- or -COO-. General methods for forming all of these linkages between aromatic systems are well known in the art.

Particularly preferred compound of Formula (2) are those wherein each R is independently OH or is 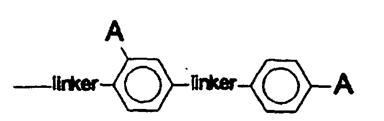 or 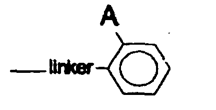 wherein linker is as defined above. Also preferred are compounds of Formula (2) wherein each n is 0 or 1, especially wherein each R is independently OH.

Also preferred are embodiments wherein the compound of Formula (2) is selected from the group consisting of 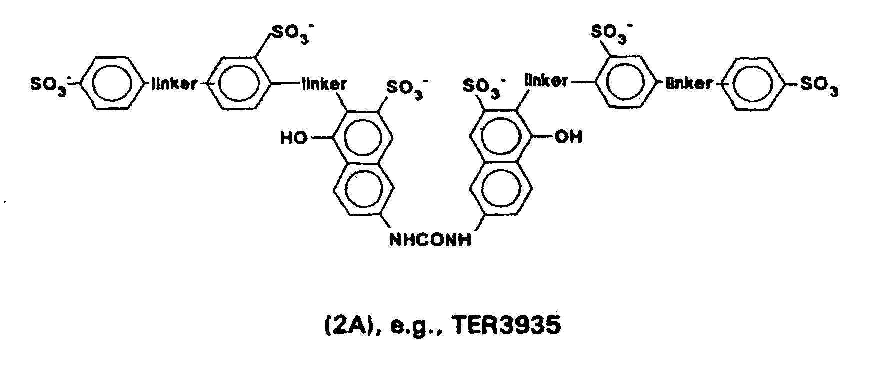 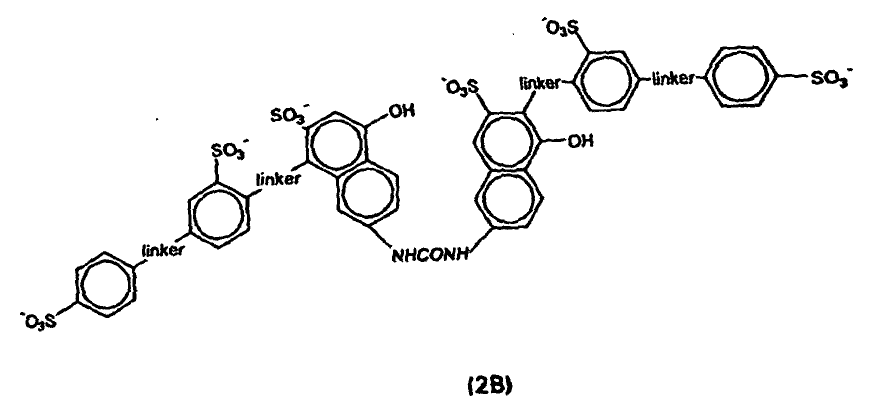 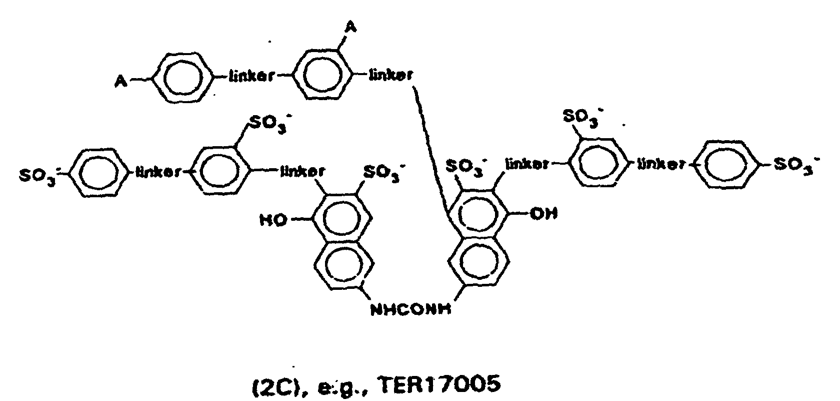 wherein each linker is independently either -N=N- or -NHCO-.

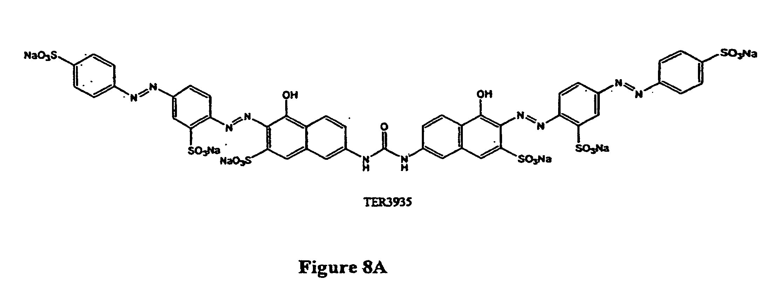

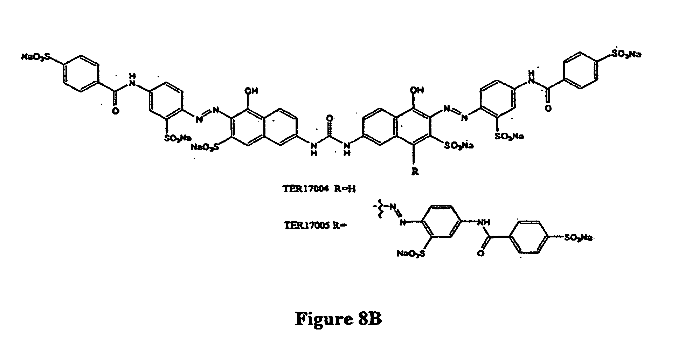

Particularly preferred are TER16998 and the compounds shown in Figures 8A and 8B herein.

Also preferred among compounds of the general Formula (1) are those of Formula (3): 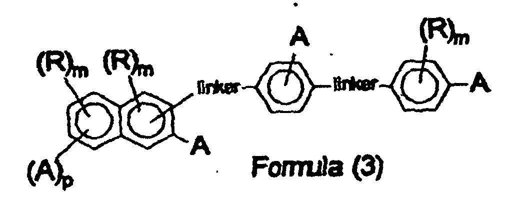 wherein each of R, A, linker and m are as defined above and
wherein p is 0 or 1.

Preferred are embodiments wherein
m is 0 or 1;
n is 0, 1, or 2; and
each linker is independently an isostere of -N=N- or of -NHCO-.

In the compounds of Formula (3), the proton-accepting substituents represented by "A" may be anionic or may be sufficiently nucleophilic to accept a proton at physiological pH. Particularly preferred embodiments of A include -SO₃X, OP(OX)₃ and -COOX where X is a hydrogen atom or a cation depending on pH. Suitable cations include inorganic cations such as sodium, potassium, calcium and the like or may be organic cations such as those provided by organic bases, for example, caffeine. Also included in embodiments of A are amino substituents including primary, secondary, and tertiary amines. Typical bioisosteres of anionic ligands such as tetrazole rings, even when they are not charged.

The noninterfering substituents on the naphthyl moieties in Formula (3) may or may not be present -- i.e., each n is independently 0, 1 or 2. The position of R is arbitrary in each case; preferred embodiments ofR include substituted or unsubstituted hydrocarbyl moieties, whether straight-chain, branched or cyclic and whether aromatic or nonaromatic. Among these are included but not limited to alkyl substituents of 1-6C, alkenyl substituents of 1-6C, and alkyl or alkenyl substituents wherein the carbon chain is interrupted by one or more heteroatoms such as O, N or S. Substituents may also be of the formula -OR', -NR'₂ and -SR', wherein R' is H or is R as defined above. Particularly preferred embodiments include -OH and additional aromatic moieties containing proton-accepting substituents.

In the compounds of Formula (3), each linker is independently an isostere of -NHCONH-, such as -NHCNHNH-, -NHCOO- or -OCOO- or may be an isostere of -N=N-, such as -CH=CH-, -CH=N- or -CH₂CH₂- or -NHCH₂-. The linkers may also be isosteres of -NHCO-, such as -OCO- or -COO-. General methods for forming all of these linkages between aromatic systems are well known in the art.

Particularly preferred compound of Formula (3) are those wherein each R is independently OH or is 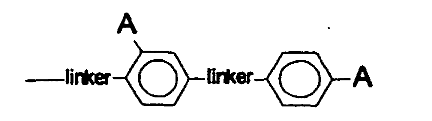 or 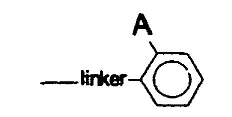 wherein linker is as defined above. Also preferred are compounds of Formula (3) wherein each n is 0 or 1, especially wherein each R is independently OH.

Also preferred are embodiments are those wherein each linker is independently either -N=N- or -NHCO-.

More preferred are compounds of the formula 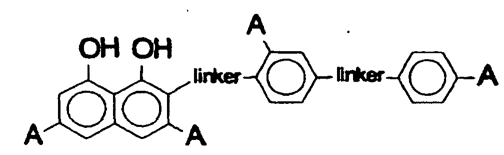 wherein each linker is independently -CH=CH- or -NHCO-.

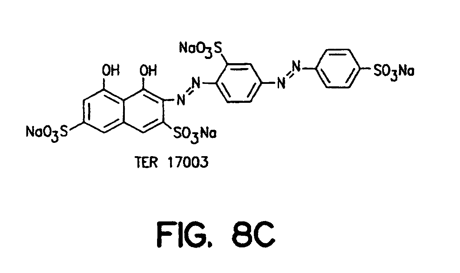

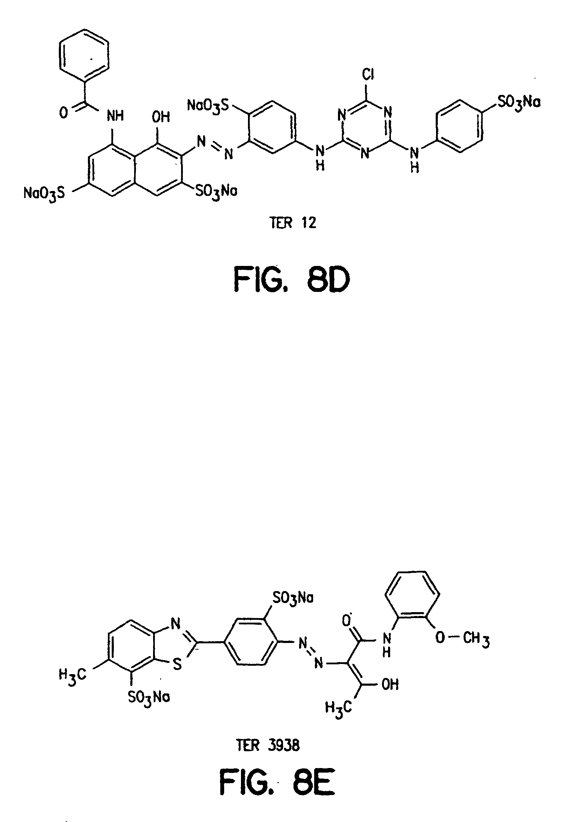

Particularly preferred are the compounds shown in Figure 8C and 8D herein.

Identification of the foregoing compounds was effected as follows: A library of compounds described in U.S. Patent No. 5,587,293, incorporated herein by reference, containing the fingerprints of 10,000 compounds obtained against a panel of 18 reference proteins, was sorted so that clusters of fingerprints with similar characteristics were grouped to select 50 representative compounds as a "training set." Each of these 50 representative compounds was tested for its ability to activate the insulin receptor. A sample believed to consist only of TER12 shown in Figure 8D, whose fingerprint did not group and was not a member of a cluster was the only tested compound that was successful in activating this receptor using the assay set forth in Example 1. Although it was later found that the component of the tested sample that had the structure of TER12 was not as active in this assay as a component present at lower concentration, Component A, the structural similarities of TER12 and Component A are evident and are sufficient to permit both Component A and TER12 to be used as fingerprint comparison standards.

The foregoing method can be generalized, in combination with the assay method described in Example 1 hereinbelow to screen for compounds which activate any receptor which undergoes autophosphorylation. In general, the method comprises identifying a compound that activates a receptor containing a kinase portion by autophosphorylation. The method comprises contacting each member of a set of maximally diverse candidate compounds with the receptor or kinase portion of the receptor and detecting the presence or absence of tyrosine phosphate on the receptor or kinase portion. A successful candidate is identified as a member of the set wherein an increased level of tyrosine phosphate as compared to basal is detected in the receptor or kinase with which it was contacted.

Chemical catalogs were searched for compounds with structural features similar to those of TER12 or which when tested had fingerprints showing essential matching features. Of 42 compounds identified as having similar substructures, four showed activity with respect to the receptor, among these was a sample containing TER3938 shown in Figure 2B, although most of the activity was later shown to be due to Component A, a structurally related component present at lower concentration. Thus, the fingerprints of these compounds identified with respect to a standard reference panel (further described hereinbelow) can be used as standards for comparison to fingerprints of candidate compounds likely to have the same activities.

U.S. Patents 5,217,869, 5,300,425 and 5,587,293, the contents of which are incorporated herein by reference, describe techniques for identifying compounds with similar properties by comparing their fingerprints. Thus, as used herein, a "characterizing fingerprint" refers to a binding profile as described in U.S. Patent No. 5,587,293 which provides sufficient information about the binding activity of a particular compound to characterize it. Typical profiles are illustrated, for example, in Figure 2C of that patent which illustrates a profile against only a nine-member panel. Higher numbers of members of the panel are preferred, however, typically 12, 16 or 18-member panels can also be used. By a "similar" binding profile is meant that the general pattern obtained for the compound with a "similar" profile is the same as the general pattern exhibited by the reference, for example, TER 16998.

Briefly, a fingerprint for a single compound (which characterizes it) is obtained by testing the binding or reactivity of the compound with respect to a reference panel of substances which may, for instance, be antibodies, proteins in general, or other substances which exhibit varied degrees of reactivity with respect to most compounds. The reference panels are chosen so that they represent virtually the totality of chemical space -- i.e., a set of substances so varied in its spatial and charge contours that the ability to react with any other substance is contained at least somewhere within the panel. Each compound reacted with the panel, then, yields a characteristic pattern of reactivities which could be considered a fingerprint. Compounds which exhibit similar fingerprints exhibit similar patterns of reactivity and properties. Thus, if a target receptor is known to bind to a specific ligand, one can identify a compound which also binds to the receptor by choosing a compound whose fingerprint is similar to that of the known ligand. Here, the fingerprints of candidates from, for example, libraries of compounds are compared to the corresponding fingerprints of TER12, TER3938 or other compounds identified as activators of the receptor, preferably Component A and TER16998. It will be noted that it is of no consequence that TER12 and TER3938 were themselves later shown to be less active in the IR kinase assays than other components contained in samples of these compounds with respect to the utility of their fingerprints for identification of compounds that have IR kinase activity since the active contaminants are chemically similar.

Because of the modular nature of the active compounds, with repeated use of similar motifs, synthesis of variants which fall within the genus set forth in Formula (1) is particularly convenient using standard combinatorial chemistry techniques. Typically, parallel synthesis of compounds with a variety of alternative building blocks used at each successive step allows numerous variants to be made quickly. Commonly, but not necessarily, the parallel operations are performed on solid-phase immobilized precursors. The resulting libraries of compounds having in common their conformity with the structure set forth as Formula (1) are useful to identify new active compounds and also to establish the pharmacologically relevant features of the active moieties which can then be further combined.

Assessment of the structural features of an individual active compound, especially those that are shared by several active compounds, in contrast, for example, to the compounds which do not activate the insulin receptor, also permits the design of suitable candidates for synthesis and testing. Methods for such analysis and identification of such structural features are known in the art. See, for example, Nesnow, S. *et al. J Toxicol Environ Health* (1988) 24:499-513, which describes the assignment of structural features among a group of 36 arylazo dyes as related to their ability to be reduced by rat liver microsomal azoreductase.

The following examples are intended to illustrate but not to limit the invention.

### Example 1

### Apparent Effect of TER 12 on Insulin Receptor Kinase Autophosphorylation

A. This assay is a modified form of that described in Hagino, H. *et al. Diabetes* (1994) 43:274-280. Briefly, human insulin receptors (hIR) were partially purified from placental extracts or from cell line IM-9. The partially purified hIRs were captured into microplate wells by incubating them for 90 minutes with wells coated with a monoclonal antibody to hIR. The wells were then treated with various dose levels of insulin and/or test compounds for 15 minutes at room temperature; ATP (10 µM) was then added to permit kinase activity to proceed. After 60 minutes, the wells were washed, and then treated for 60 minutes with biotinylated antibody directed against phosphotyrosine (PY-20) and unbound materials again washed away. The wells were then incubated with a conventional streptavidin peroxidase system for 30 minutes to assess the level of phosphorylated tyrosine.
   When tested in this assay, insulin gave a dose response curve showing an EC₅₀ of about 0.3 nM and a maximal activity at about 100 nM. The EC₅₀ is similar to that obtained for binding of labeled insulin to various cells and tissues.
   Two sets of compounds of 50 members each, selected to be maximally diverse, as defined in U.S. Patent 5,300,425 and incorporated herein by reference, were screened in the foregoing assay. Of these 100 compounds, only a sample composed mainly of TER12 (see Figure 8C) showed apparent agonist activity. In the absence of insulin, 20 µM of this sample stimulated autopbosphorylation over five-fold (0.3 nM insulin stimulates phosphorylation approximately to this extent). Thus, the activity of insulin at approximately 0.3 nM is roughly equivalent to that shown by this sample at approximately 20 µM and a component of this sample shows the ability directly to stimulate autophosphorylation.
   In addition, the sample enhanced the ability of insulin to stimulate autophosphorylation. The addition of 60 µM sample to hIR contacted with 0.3 nM insulin resulted in an increase in phosphorylation of approximately three-fold and to the maximal level shown by insulin stimulation at higher concentrations. The EC₅₀ for this effect (enhancing insulin stimulation) was shown in additional experiments to be approximately 20 µM of sample calculated as TER12. These results were also confirmed by Western blot assay.
B. In an additional demonstration of the activity of a component in the sample containing TER12, the artificial substrate, poly(Glu₄Tyr) was used as the substrate for phosphorylation. Incorporation of labeled phosphate from γ-labeled ATP was measured following activation of receptor prepared as in paragraph A. About 20 µM of sample calculated as TER12 provided 75% of maximal insulin-stimulated activity; it also enhanced the ability of 0.5 nM and 5.0 nM insulin to effect phosphorylation; 0.5 nM insulin alone showed 60% maximal phosphorylation; addition of 20 µM of the TER12 sample increased this to 120%; in the presence of 5 nM insulin phosphorylation rose from 95% of maximum to 140%.
C. When tested with respect to insulin receptor agonist activity on whole cells, i.e., on the human lymphocytic cell line IM-9, the sample containing TER12 retained its ability to stimulate the receptor. In this assay, 2 x 10⁷ cells were treated with and without this sample and with and without insulin for 5 minutes, followed by three washes in isotonic medium to remove the sample containing TER12. The cells were then lysed in 0.5% Tween 20 and lysates analyzed in an ELISA assay as described in paragraph A, without the steps of incubation with ATP. After 5 minutes exposure to sample containing 20 µM TER12, basal insulin receptor kinase activity was increased two-fold and insulin stimulated insulin receptor kinase activity was increased five-fold.
D. The assay described in paragraph B was conducted by substituting, for the human insulin receptor, a recombinantly produced β chain lacking the insulin-binding domain (supplied by Stratagene, Inc.). The ability of this kinase to phosphorylate a substrate peptide (Raytide from Oncogene Sciences) is stimulated by TER12 at 25 µM. (In addition, a known inhibitor believed to act at the ATP site on the kinase also inhibits this modified form of the receptor.)
E. Insulin is able to induce the differentiation of 3T3-L 1 fibroblast cells to an adipocyte-like morphology as measured by Oil Red O uptake. The sample containing TER12 alone does not appear to effect differentiation; however, at a concentration of 20 µM it enhances the differentiating effect of insulin. This activity is similar to that exhibited by pioglitazone described above. Insulin also enhances glucose transport in this cell line. Again, the sample alone failed to stimulate glucose transport significantly, but enhanced the ability of insulin to do so.

### Example 2

### Additional Compounds with TER12-Like Activity

Using substructure searching based on the TER12 molecule, 42 candidate compounds were obtained and assayed according to the procedure of Example 1,

A sample containing TER3938, shown in Figure 8E, also showed agonist activity. TER3938, shown in Figure 8E and known as Direct Yellow No. 27, showed an EC₅₀ of 8 µM in this *in vitro* assay; it also enhanced the activity of insulin in stimulating autophosphorylation of insulin receptor on intact IM-9 cells. In addition, a sample containing TER3935, shown in Figure 8A, was active in the IR kinase assay.

### Example 3

### Identification of an Active Component of TER12 and TER3938-Containing Samples

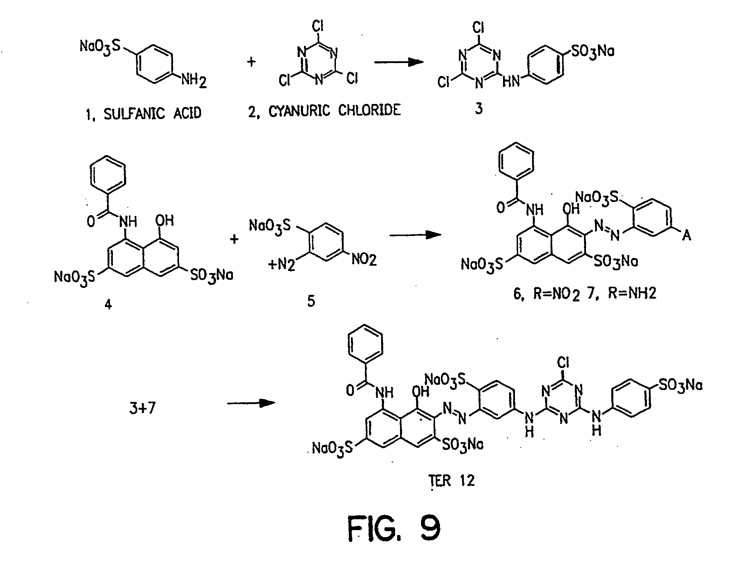

TER1 2 was synthesized by the reaction scheme shown in Figure 9. TER12 synthesized using this scheme, and TER12 when extensively purified from commercial sources were active in the assays set forth in Example 1.

In addition, the sample containing TER3938, also obtained from commercial sources, when purified to 95% purity by reverse-phase HPLC, retained its activity; however, when this sample was washed with aqueous sodium carbonate, the insoluble compound shown in Figure 8E as TER3938 was less active in the IR kinase assay; the aqueous layer, however, retained full activity. These results led to the conclusion that some of the activity shown in samples purportedly containing only TER12 and TER3938 was due to a minor component. This minor component was postulated to be Component A. Component A, obtained from commercial sources, was purified by C-18 reverse-phase preparative HPLC and retained its activity in the IR kinase assay. Component A was subsequently demonstrated to be a minor component in samples containing both TER12 and TER3938. No Component A was found in TER3935 which is active after extensive purification.

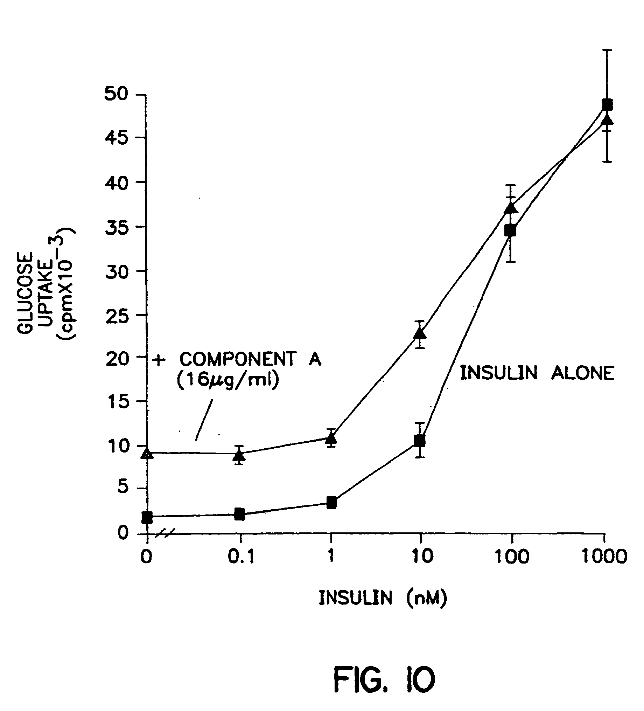

Component A, purified from a commercially supplied sample, enhances glucose uptake in differentiated 3T3-L I cells, and the activity is not dependent on the presence of insulin. It is, however, dependent on the activity of PI-3 kinase, confirming that the glucose uptake is mediated via the insulin signaling pathway. The ability of 16 µg/ml concentrations of Component A to enhance glucose uptake at various insulin concentrations is shown in Figure 10.

In the assay, 3T3-L1 pre-adipocytes were induced to differentiate into adipocyte morphology using standard protocols. Five days after induction, the cells were treated with 16 µg/ml of Component A in the presence of various levels of insulin for 30 minutes. Glucose uptake was measured using ¹⁴C glucose as label. As shown, 16 µg/ml of Component A alone effects uptake at approximately the level shown by 20 µM concentrations of insulin in the absence of this concentration of Component A.

### Example 4

### Additional Compounds Related to TER3935

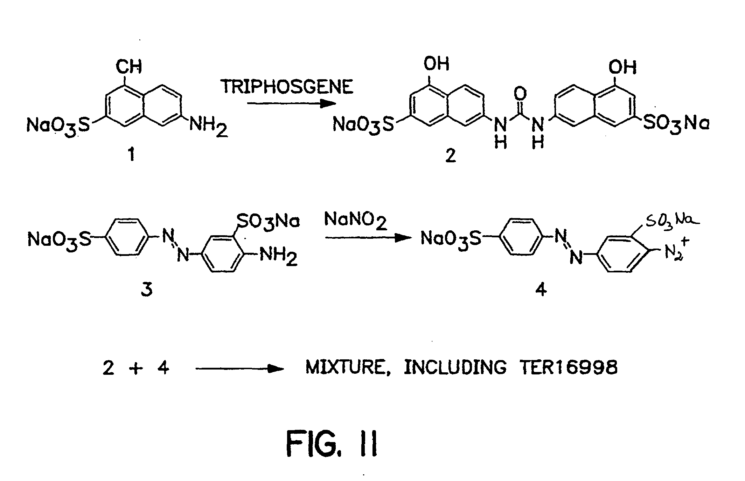

An additional compound with a structure regioisomeric to that of TER3935, TER 16998, was isolated by preparative reverse-phase chromatography from the reaction mixture produced by the synthetic scheme shown in Figure 11. Spectral data confirm that the isolated compound was of the formula shown in Figure 5A.

TER16998 activates the insulin receptor kinase directly, enhances autophosphorylation and substrate phosphorylation mediated through the insulin receptor, potentiates glucose transport and lowers blood glucose in the db/db mouse model of diabetes. These results were obtained as follows:

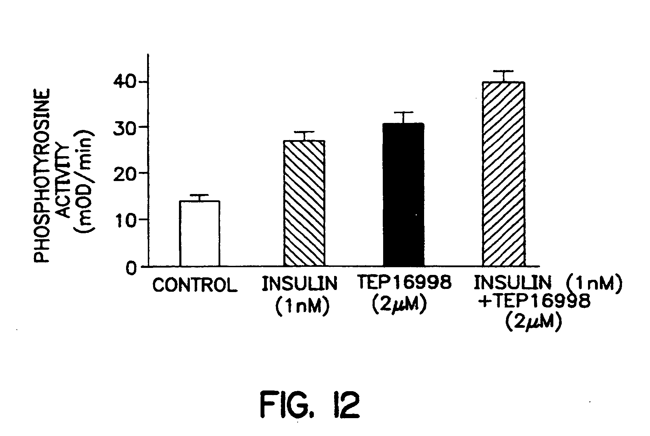

The assay described in Example 1, paragraph A, was conducted with a control lacking any additions, in the presence of insulin alone at 1 nM, in the presence of TER16998 at 2 µM, and in the presence of a combination of these components at the stated concentrations. As shown in Figure 12, TER16998 alone is able to activate autophosphorylation of the receptor at this concentration, as well as to potentiate the effect of insulin.

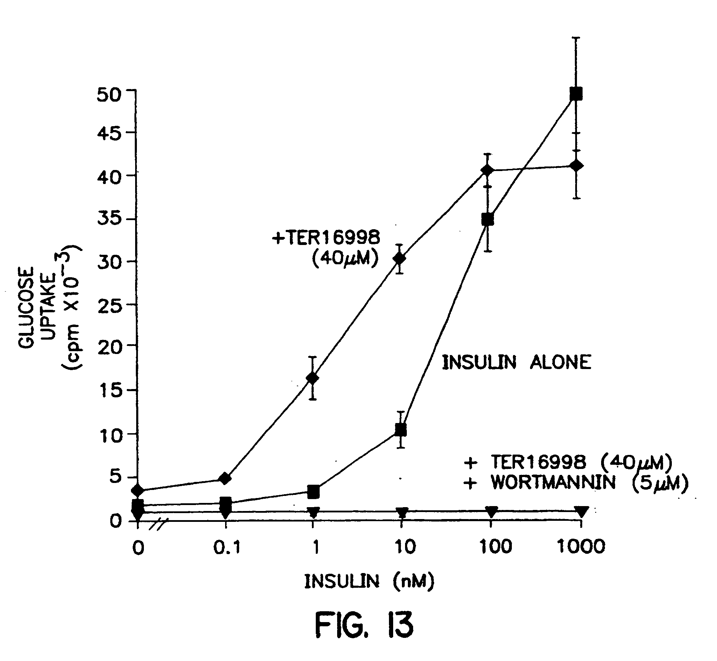

In addition, in an assay for glucose uptake by 3T3-L1 adipocytes, described in Example 3, TER16998 produced an acute effect sensitizing the cells to insulin. This was inhibited, as expected, by 5 µM wortmannin which inhibits PI-3 kinase, confirming that TER16998 exerts its effect through the insulin-signaling pathway. These results are shown in Figure 13. As shown, 40 µM of TER 16998 potentiates the effect of insulin at a range of concentrations.

Significantly, TER16998 was not able to stimulate the phosphorylation activity of epidermal growth factor receptor in an EGF receptor kinase assay.

The effect of TER16998, of Component A, and of insulin on the distribution of the Glut4 transporter in 3T3-L I adipocytes was determined by incubating the cells for 15 minutes with insulin or one of these compounds, after which the cells were fixed and stained with an anti-Glut4 antibody followed by FITC-conjugated secondary antibody. The results were visualized under a fluorescent microscope. The results showed that insulin and Component A produce a dramatic redistribution of Glut4 to the membrane surfaces whereas in untreated cells a diffuse pattern is obtained. TER16998 has a similar effect but less dramatic than that of insulin or Component A.

### Example 5

### Effect of TER16998 in Diabetic Mice

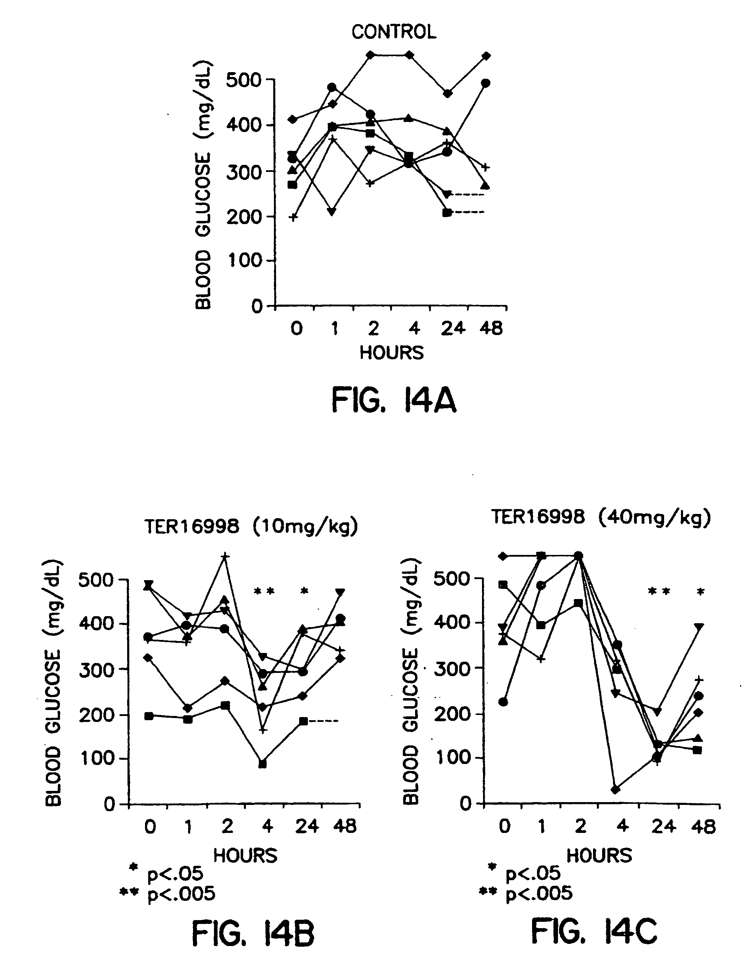

Mice which are standard models of Type II diabetes, db/db mice, were permitted to eat *ad libitum* and were administered TER16998 at 10 mg/kg and 40 mg/kg, or a vehicle as a control. Figure 14 shows the effect of this compound on the concentration of glucose in the blood of these animals. As shown in Figure 14, 10 mg/kg to some extent and 40 mg/kg to an appreciable extent decrease blood glucose over a period of 24 hours from the time of administration.

### Example 6

### Synthesis of Invention Compounds

In general, the polymeric aromatic compounds of the invention are synthesized using either solution-phase or solid-phase-based syntheses. For solid-phase synthesis, the monomers are, for example, coupled to a phenolic resin of the Formula (10) shown in Figure 9. This phenolic resin is prepared by oxidation of the corresponding boronic ester polystyrene resin described by Farrall, M.J. and Frechet, J.M.J. *J Org Chem* (1976) 41:3877. The phenolic resin is condensed with an initial monomer, 11, as shown. The condensation product, 12, is treated with N-butyl lithium to replace the bromonium ion with lithium and this intermediate is condensed with a monomer, for example, of the Formula (13), to provide the solid-supported dimer, 14. The linking -CHOH- can be, if desired, reduced to -CH₂-. Subsequent condensations in the presence of formaldehyde and concentrated sulfuric acid result in polymers of the desired length which can then be removed from the resin in sodium methoxide. This process is outlined in Figure 9.

The synthesis can be varied by altering the nature of the monomer or dimer added to the supported starting group.

Synthesis of suitable oligomers is also conducted in solution phase. In one set of reactions, the synthesis is a variant of that described by Arduini, A. *et al*. *Tetrahedron* (1990) 46:3607-3613. Intermediate dimers or trimers are obtained by condensing an aromatic aldehyde with an aromatic bromide and further condensation is effected in the presence of formaldehyde and sulfuric acid. Dimers shown as Formulas 18,19 and 20, which contain the proton-accepting substituent in all possible configurations can be obtained by condensation of the appropriate naphthyl lithium with a naphthyl formyl derivative obtained by treating the naphthyl lithium with dimethyl formamide (DMF). If desired, compounds 18, 19 and 20 can be reduced with HSiEt₃/TFA to the corresponding methylene-bridged dinaphthylenes. 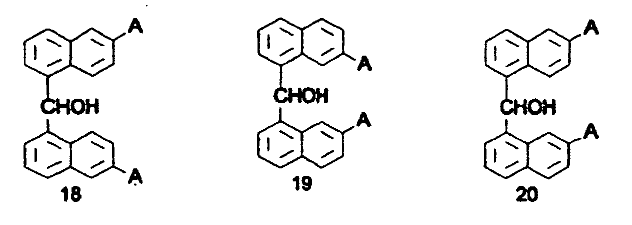 The dibromo dimer of the form 23 is obtained as described by Arduini, A. (*supra*) by condensing the corresponding naphthyl bromide with formaldehyde in the presence of sulfuric acid.

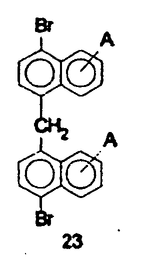

The corresponding trimers can be obtained by treating such bromo-substituted dimers with N-butyl lithium and then with an aldehyde of the Formula 21 or 22.

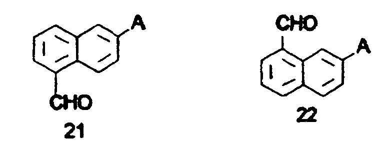

The oligomers may also be extended by coupling naphthyl or other aromatic moieties with dialdehydes, for example, 4,4'-biphenyldicarboxaldehyde or terephthaldehyde.

By appropriate choice of the position of the proton-accepting substituent A, and of the reactive substituents on the aromatic moieties, the desired oligomers may be synthesized.

As set forth above, the polymer of the formula 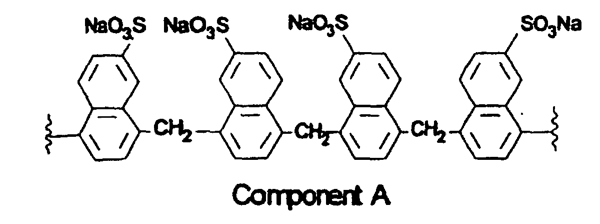 is active in the insulin receptor kinase assay described above and exhibits the ability to potentiate insulin activation and glucose uptake.

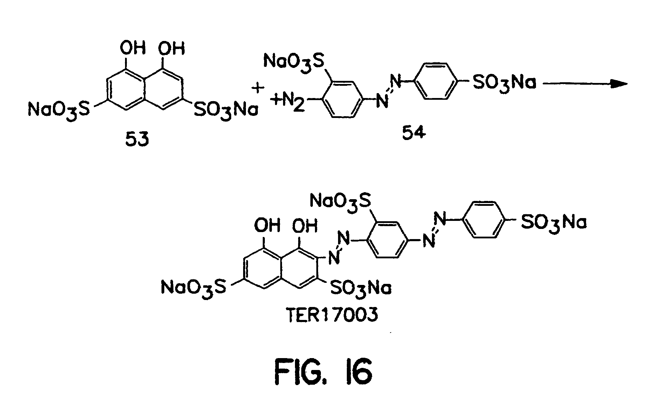

Compound TER17003 is synthesized as shown in Figure 16.

TER17003 was tested in the IR kinase assay set forth in Example 1, paragraph A, and found to be active in this assay.

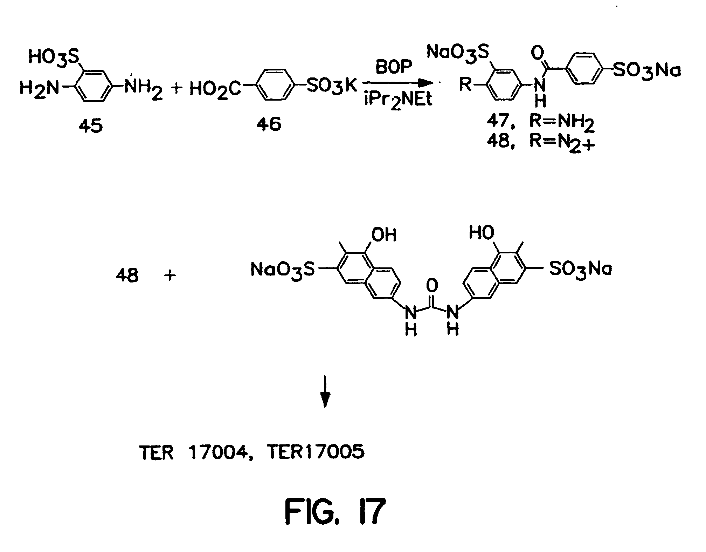

Compounds TER17004 and TER17005 replace certain azo-linkers of TER3935 with the corresponding amide linkers. These compounds are synthesized as shown in Figure 17.

The resulting compounds, TER17004 and TER17005, were tested in the IR kinase assay set forth in Example 1, paragraph A, and found to be active in this assay.

### Example 7

### Peptides which Compete with Receptor for Binding to TER16998

In order to identify additional regions of the CKD to which TER16998 binds, a series of 14 peptides (**ryan peptides") were synthesized. These peptides were chosen to correspond to distinct surface elements of the CKD made evident by the X-ray structure. Collectively, the 14 peptides cover 85% of the surface-exposed residues. These peptides were tested for their ability to inhibit the activation of insulin receptor by TER16998. The assays were conducted as follows:

The reaction mixture contains 10 µl of 50 mM Tris, pH 7.5,100 mM NaCl, 8 mM MgCl₂, 2 mM MnCl₂, 2 mM DTT, 9 µM human IR-CKD, 4 mM ATP, and 14.5 µM TER16998. Control mixtures contain no peptides; peptides tested were present at 20 µM or at 100 µM. The reactions were incubated for 5 or 15 minutes and then stopped with gel sample buffer containing EDTA to 50 mM final concentration. Samples were loaded onto a 10% native polyacrylamide gel and run for 2 hours at 15 mA. Gels were stained with Coomassie blue and the degree of autophosphorylation measured by comparison to control lanes using the band positions in the native gel as an indication of phosphorylation.

Of the 14 peptides, only 3 were found able to diminish the activation effect of TER16998. These peptides were:
"ryan 3" which extends from Met1120 to Asn1137 and thus corresponds to the catalytic site floor at the end of helix E;
"ryan 6" which extends from Pro1235 to Thr1252, i.e., helix H and helix I; and
"ryan 9" which extends from Arg1089 to Thr1105.

Table 1 below sets forth the location and amino acid sequence of the 14 peptides prepared.

**Table 2**

| **Ryan No.** | **Region** | **Sequence** |
|---|---|---|
| 1 | Asn1033-Thr1055 | NESASLRERIEFLNEASVMKGFT |
| 2 | Met1079-Pro1099 | MAHGDLKSYLRSLRPEAENNP |
| 3 | Met1120-Asn1137 | MAYLNAKKFVHRDLAARN |
| 4 | Glu1201-Gly1225 | EITSLAEQPYQGLSNEQVLKFVMDG |
| 5 | Asn1014-Ala1028 | NARDIIKGEAETRVA |
| 6 | Pro1235-Thr1252 | PERVTDLMRMCWQFNPKM |
| 7 | Ser992-Ser1006 | SREKITLLRELGQGS |
| 8 | His1057-Gln1070 | HHVVRLLGVVSKGQ |
| 9 | Arg-1089-Thr1105 | RSLRPEAENNPGRPPPT |
| 10 | Gly1100-Gln1111 | GRPPPTLQEMIQ |
| 11 | Gly1163-Gln1174 | RKGGKGLLPVR |
| 12 | Pro1178-Ser1190 | PESLKDGVFTTSS |
| 13 | Gln1217-Asn1233. | QVLKFVMDGGYLDQPDN |
| 14 | Arg1253-Ser1270 | RPTFLEIVNLLKDDLHPS |

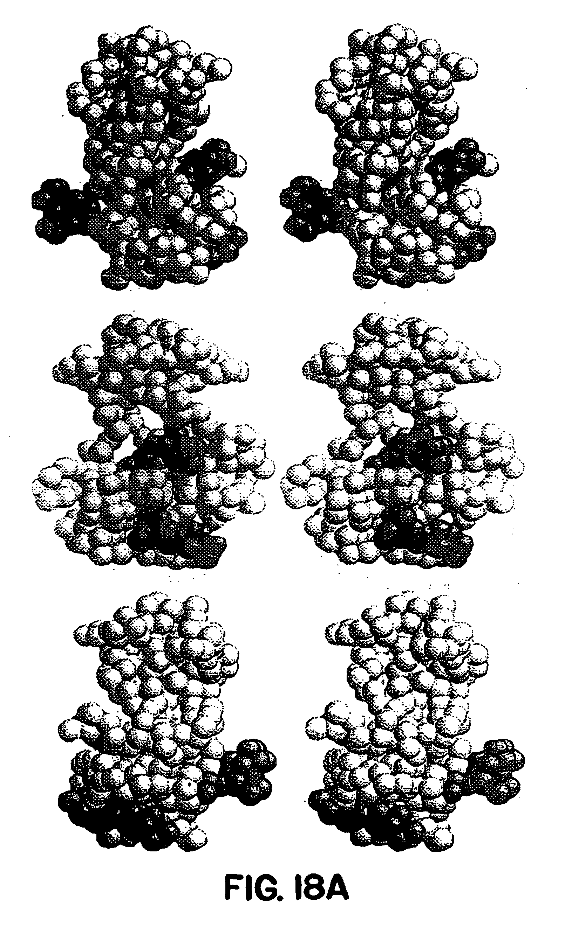

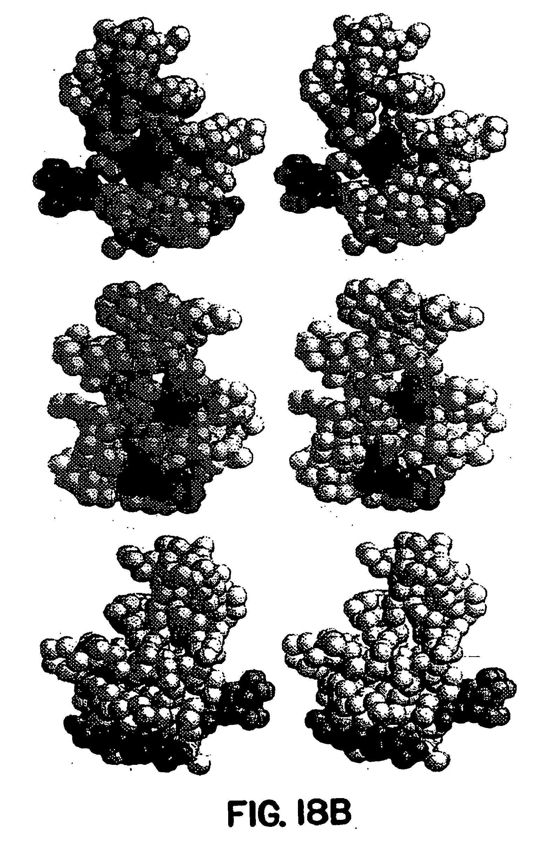

The location of the active peptides in terms of the three-dimensional structure of the CKD is shown in Figures 18A and 18B. Figure 18A shows the location of the peptides in the basal state; Figure 18B shows the position of these peptides when the receptor is activated. It is seen that these peptides form a rough "belt" around the receptor.

### Example 8

### Cell Penetration of TER16998

In order to assess whether TER16998, which potentiates insulin stimulation of the insulin receptor when whole cells are used in the assay, is believed to be effective through its ability to enter the cells. This is established in the protocol described below:

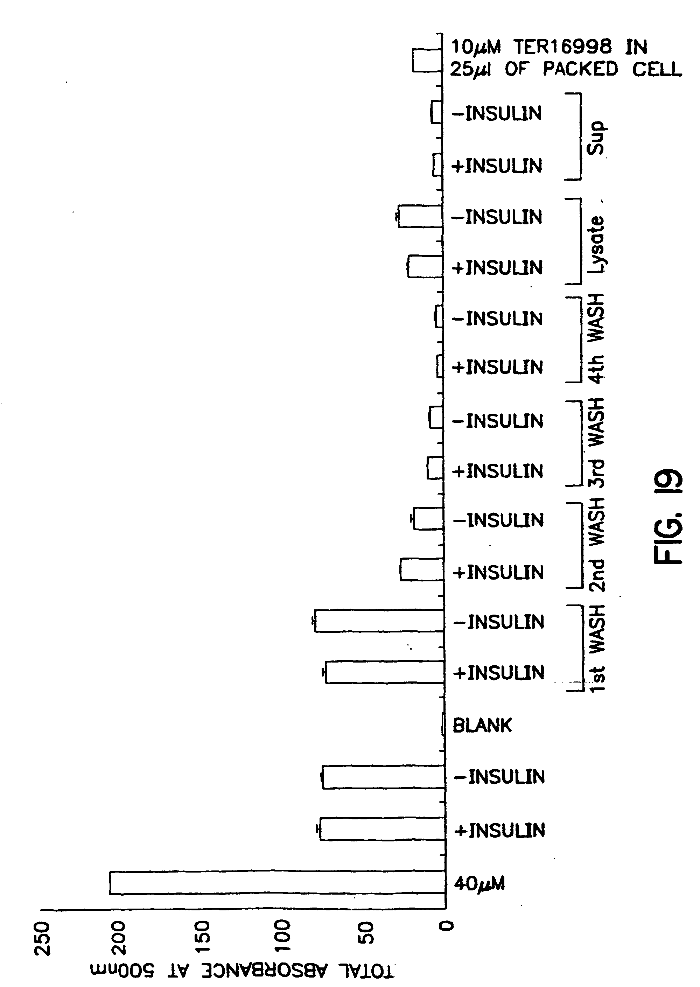

JM-9 cells were treated with TER16998 (40 µM) in the presence and absence of insulin and then washed repeatedly with 10% fetal bovine serum in PBS in order to remove any TER16998 which adheres to the cells. When no further TER16998 was detectable in the washes, the cells were lysed and the compound was found present in the lysate by spectrophotometry. These results are shown in Figure 19.

As shown, TER16998 was found in the lysate. Thus, it is clear that this compound can enter the cells and directly interact with the CKD region.

### Example 9

### Ability of TER16998 to Enhance the Effect of Insulin on Blood Glucose Levels

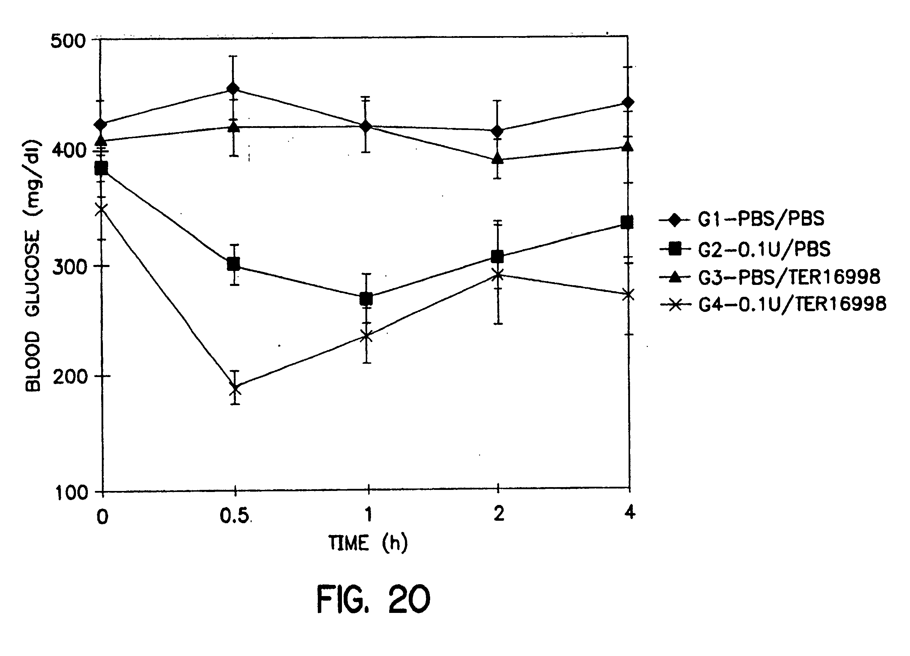

In this study, glucose uptake in diabetic db/db mice was determined in the presence and absence of insulin and in the presence and absence of 10 mg/kg TER16998. In this example, unlike Example 5, the animals were fasted. In the absence of insulin, glucose remained at high levels in the blood. In the presence of insulin, as expected, glucose levels dropped but began to rise again after about one hour. In the presence of TER16998, however, glucose levels were lowered more drastically and had not recovered to the levels shown in mice treated with insulin alone after 4 hours. These results are shown in Figure 20 where the solid diamonds and triangles indicate mice treated with a placebo or with TER16998 alone, respectively. The solid squares show blood glucose levels for mice treated with insulin alone and the "X" data show blood glucose levels for mice treated both with insulin and TER16998.

## Claims

1. An *in vitro* method to modulate the kinase activity of insulin receptor and/or to potentiate the insulin activation of insulin receptor and/or to potentiate the stimulation by insulin of cellular glucose uptake or to stimulate the uptake of glucose in cells displaying the insulin receptor which method comprises contacting said insulin receptor or the kinase portion thereof or said cells with a compound which comprises the Formula 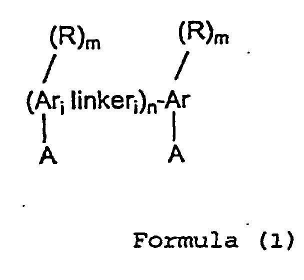 wherein:
each Ar is independently an aromatic moiety selected from benzene, naphthalene, pyridine, quinoline or benzothiazole;
each A is independently -SO₃X, OP(OX)₃, -COOX, where X is a hydrogen atom or a cation;
each R is independently a substituted or unsubstituted hydrocarbyl moiety, branched or unbranched, cyclic, aromatic or nonaromatic, wherein unbranched hydrocarbyl chains may be interrupted by one or more heteroatoms of O, N, or S; or each R is independently OR', NR'₂ or SR', wherein R' is H or R as defined above;
m is 0, 1 or 2; n is 1-6; and
each linker is independently -CH₂-, -N=N-, -CH=CH-, -NHCO- or -NHCONH- or an isostere thereof, wherein when n is 1, at least one Ar must comprise at least 2 fused aromatic rings.

2. The method of Claim 1 wherein the compound of Formula (1) has the Formula 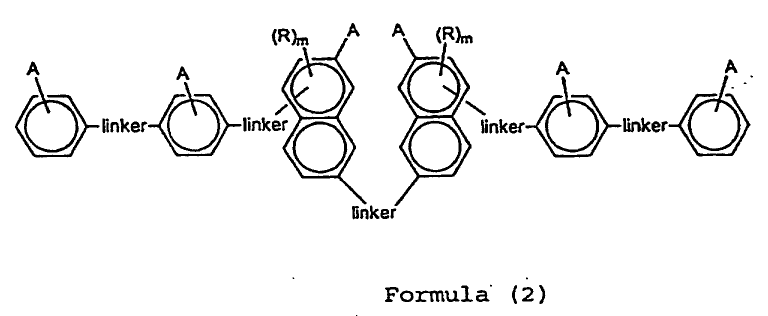 wherein each R, A, linker, and m are as defined in Claim 1.

3. The method of Claim 1 wherein the compound of Formula (1) has the Formula 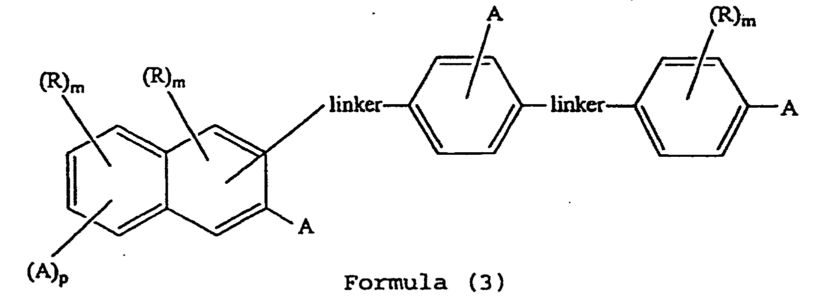 wherein each of R, A, linker and m are as defined in Claim 1 and wherein p is 0 or 1.

4. The method of Claim 1 wherein m is 0 or 1, n is 4-6, and each linker is independently -CH₂-, -CH=CH, or -NHCO- or an isostere thereof.

5. The method of Claim 1 wherein said compound is of the formula 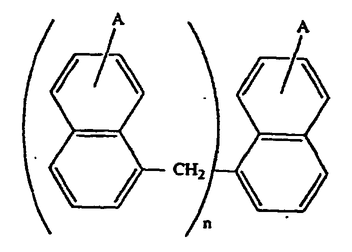 wherein n is 4-6 and A is as defined in Claim 1.

6. The method of Claim 2 or 3 wherein each R is independently OH or 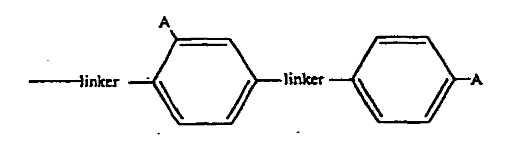 or 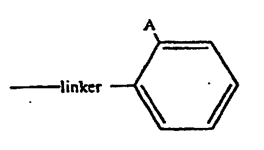 wherein A and linker are as defined in Claim 1.

7. The method of Claim 2 or 3 wherein m is 0 or 1 and each R is independently OH.

8. The method of Claim 1 or 4 wherein each R is independently alkyl (1-6C).

9. The method of any of Claims 1-8 wherein each A is independently -SO₃X or -COOX, wherein X is H or a cation.

10. The method of any of Claims 1-9 wherein all m are 0.

11. The method of Claim 3 wherein said compound is of the formula 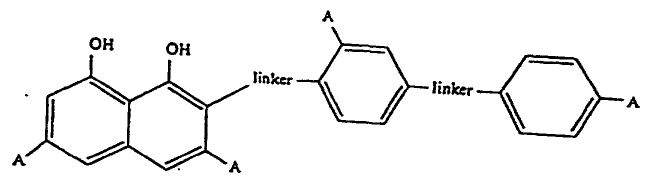 wherein each linker is independently either -NHCO-or -CH=CH-, and each A is independently -SO₃X or -COOX wherein X is H or a cation.

12. The method of Claim 2 wherein the compound is selected from the group consisting of 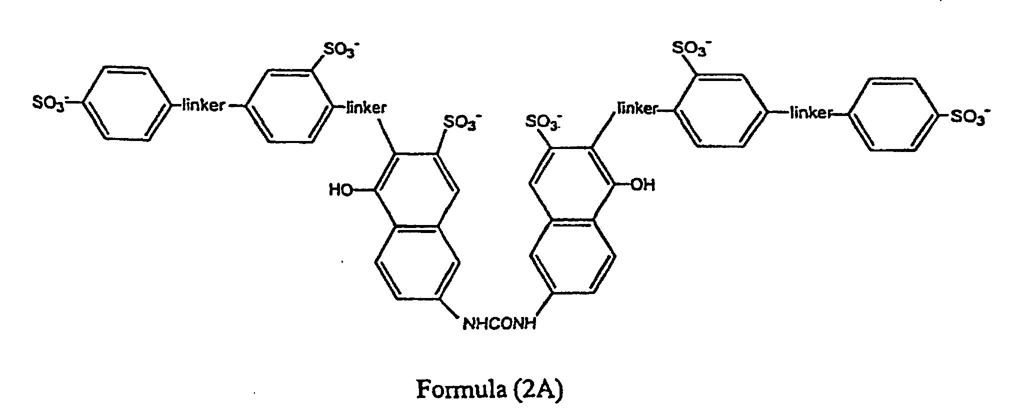 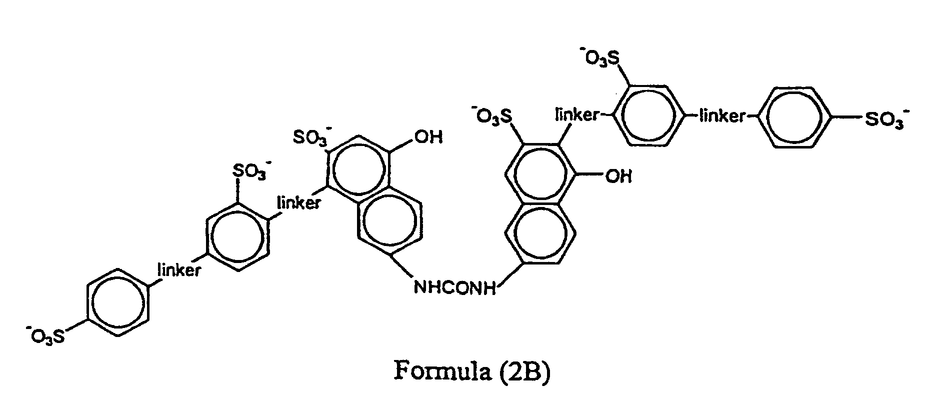 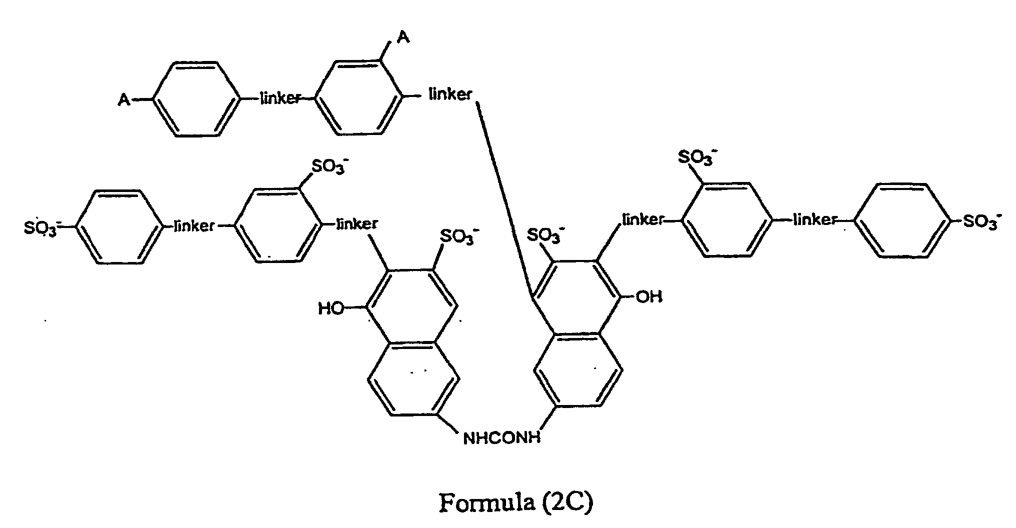 wherein each linker is independently either -N=N- or -NHCO-.

13. A method to design and synthesize a molecule that activates the insulin receptor which method comprises:
assessing an activator which is the compound of any of Claims 1-12 for structural features which correlate with said activities; synthesizing a compound containing these structural features; and testing said compound for its ability to activate the insulin receptor to verify it as an activator.

14. A method to screen candidate compounds for ability to activate the insulin receptor, which method comprises:
obtaining a fingerprint of each candidate with respect to a reference panel;
obtaining a fingerprint of an activator which is the compound of any of Claims 1-12 with respect to the same reference panel;
comparing the fingerprint of each candidate with that of said activator; and
identifying as the successful candidate a compound whose fingerprint resembles that of said activator.

15. The compound of the formula

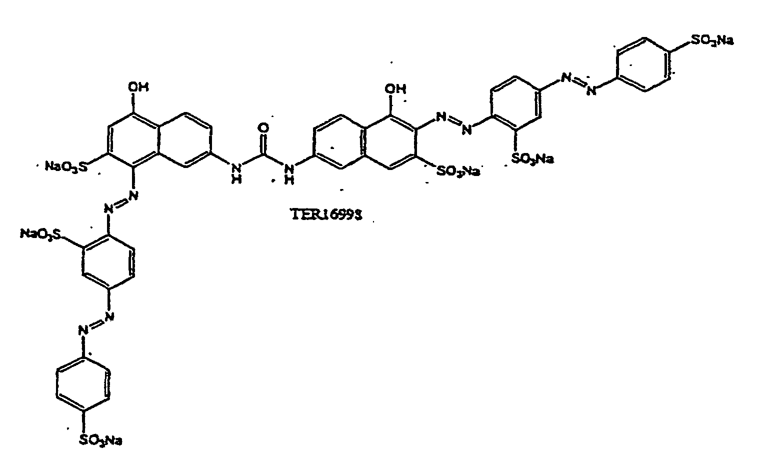

16. A composition comprising the compound of claim 15 or a pharmaceutically acceptable salt thereof and a pharmaceutically acceptable excipient.

## Patentansprüche

1. In-vitro-Verfahren zur Modulation der Kinaseaktivität des Insulinrezeptors und/oder zur Verstärkung der Insulinaktivierung des Insulinrezeptors und/oder zur Verstärkung der Stimulierung der zellulären Glukoseaufnahme durch Insulin oder zur Stimulierung der Aufnahme von Glucose in Zellen, die den Insulinrezeptor darstellen, wobei das Verfahren das Kontaktieren des Insulinrezeptors oder von dessen Kinaseanteil oder der Zellen mit einer Verbindung umfasst, die die folgende Formel umfasst: 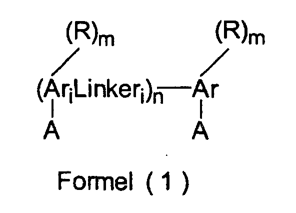 worin:
jedes Ar unabhängig ein aromatischer Bestandteil ist, gewählt aus Benzol, Naphthalin, Pyridin, Chinolin oder Benzothiazol;
jedes A ist unabhängig -SO₃X, OP(OX)₃, -COOX, worin X ein Wasserstoffatom oder ein Kation ist;
jedes R ist unabhängig ein substituierter oder unsubstituierter, verzweigter oder unverzweigter, cyclischer, aromatischer oder nicht-aromatischer Hydrocarbylbestandteil, worin nicht-verzweigte Hydrocarbylketten durch ein oder mehrere Heteroatome unterbrochen sein können, nämlich O, N oder S; oder jedes R ist unabhängig OR', NR'₂ oder SR', worin R' H oder R, wie oben definiert, ist;
m ist 0, 1 oder 2; n ist 1 bis 6; und
jeder Linker ist unabhängig -CH₂-, -N=N-, -CH=CH-, -NHCO- oder -NHCONH- oder ein Isoster davon, wobei wenn n 1 ist, mindestens ein Ar mindestens 2 fusionierte aromatische Ringe umfasst.

2. Verfahren gemäss Anspruch 1, wobei die Verbindung der Formel (1) die folgende Formel aufweist: 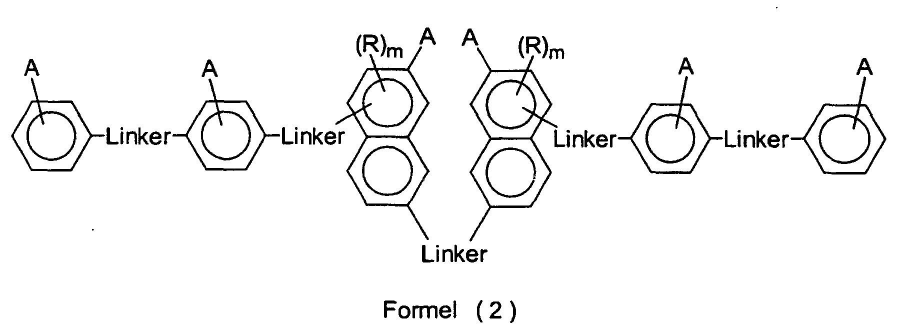 worin jedes R, A, Linker und m wie in Anspruch 1 definiert sind.

3. Verfahren gemäss Anspruch 1, wobei die Verbindung der Formel (I) die folgende Formel aufweist: 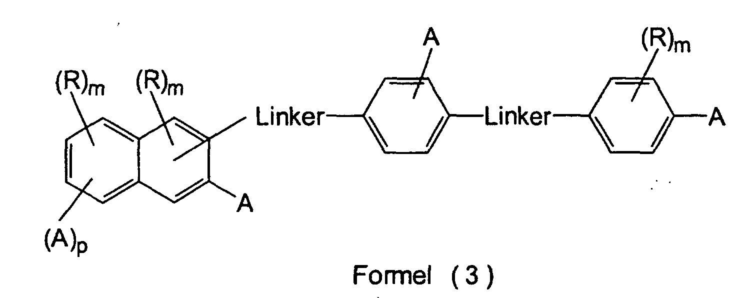 worin jedes R, A, Linker und m wie in Anspruch 1 definiert sind und worin p 0 oder 1 ist.

4. Verfahren gemäss Anspruch 1, worin m 0 oder 1 ist, n ist 4 bis 6 und jeder Linker ist unabhängig -CH₂-, -CH=CH- oder -NHCO- oder ein Isoster davon.

5. Verfahren gemäss Anspruch 1, wobei die Verbindung die folgende Formel aufweist: 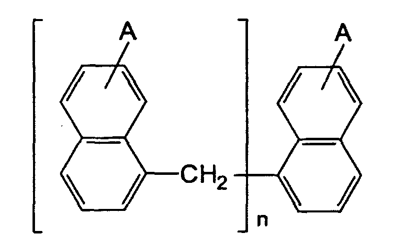 worin n 4 bis 6 und A wie in Anspruch 1 definiert ist.

6. Verfahren gemäss Anspruch 2 oder 3, worin jedes R unabhängig OH oder 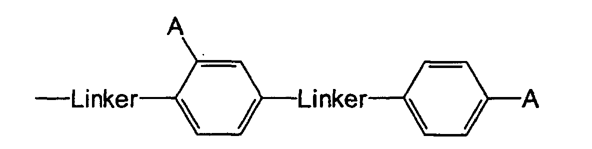 oder 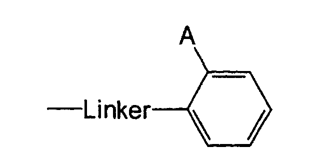 ist, worin A und Linker wie in Anspruch 1 definiert sind.

7. Verfahren gemäss Anspruch 2 oder 3, worin m 0 oder 1 und jedes R unabhängig OH ist.

8. Verfahren gemäss Anspruch 1 oder 4, worin jedes R unabhängig Alkyl (1-6C) ist.

9. Verfahren gemäss einem der Ansprüche 1 bis 8, worin jedes A unabhängig -SO₃X oder -COOX ist, worin X H oder ein Kation ist.

10. Verfahren gemäss einem der Ansprüche 1 bis 9, worin alle m 0 sind.

11. Verfahren gemäss Anspruch 3, wobei die Verbindung die folgende Formel aufweist: 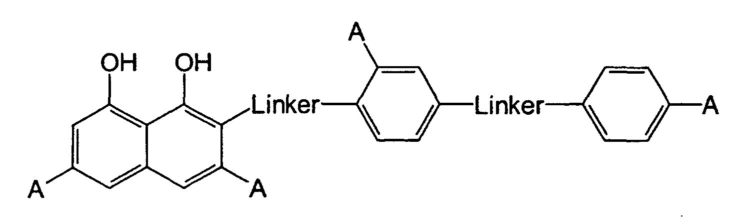 worin jeder Linker unabhängig entweder -NHCO- oder -CH=CH- ist, und jedes A ist unabhängig -SO₃X oder -COOX, worin X H oder ein Kation ist.

12. Verfahren gemäss Anspruch 2, wobei die Verbindung gewählt ist aus der Gruppe bestehend aus 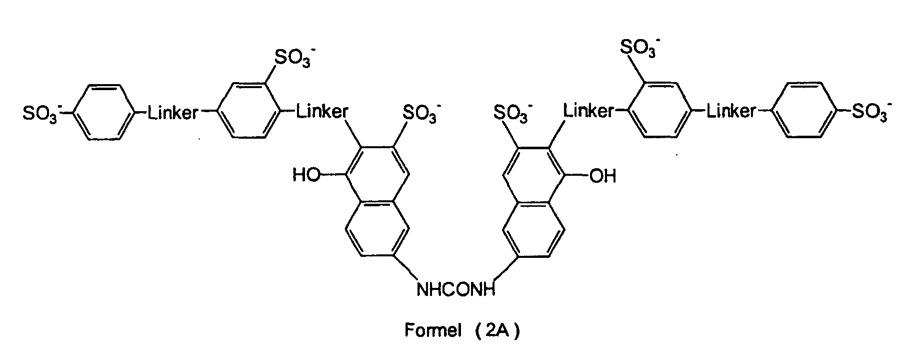 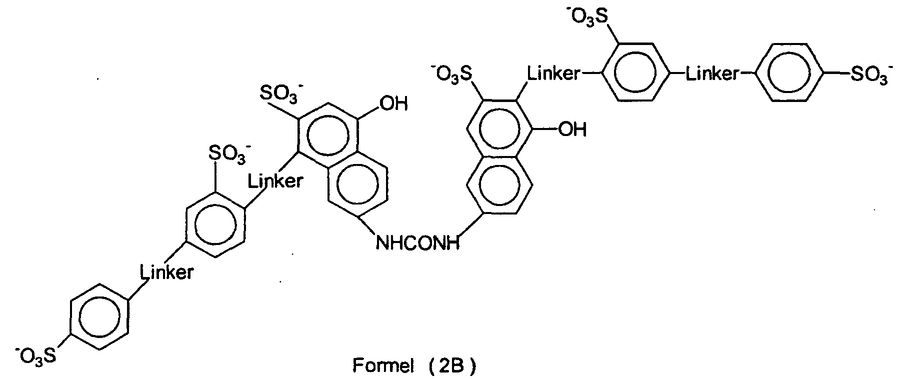 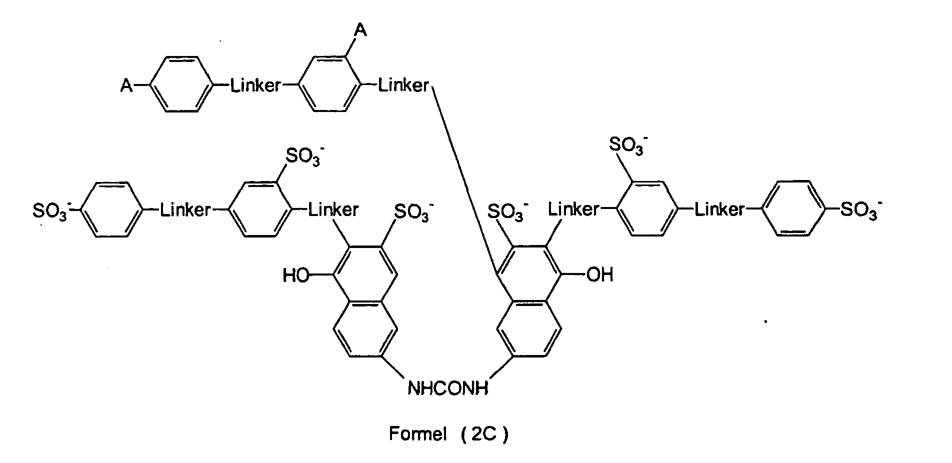 worin jeder Linker unabhängig entweder -N=N- oder -NHCO- ist.

13. Design- und Syntheseverfahren eines Moleküls, das den Insulinrezeptor aktiviert, wobei das Verfahren folgendes umfasst:
Beurteilung eines Aktivators, bei dem es sich um eine Verbindung gemäss einem der Ansprüche 1 bis 12 handelt, im Hinblick auf strukturelle Merkmale, die mit den Aktivitäten korrelieren; Synthese einer Verbindung, die diese strukturellen Merkmale enthält, und Überprüfung der Verbindung im Hinblick auf ihre Fähigkeit zur Aktivierung des Insulinrezeptors zur Sicherstellung, dass es sich um einen Aktivator handelt.

14. Screeningverfahren für Kandidatenverbindungen im Hinblick auf ihre Fähigkeit zur Aktivierung des Insulinrezeptors, wobei das Verfahren folgendes umfasst:
Erhalt eines Fingerabdrucks von jedem Kandidaten im Hinblick auf ein Referenzpanel;
Erhalt eines Fingerabdrucks eines Aktivators, bei dem es sich um eine Verbindung gemäss einem der Ansprüche 1 bis 12 handelt, im Hinblick auf dasselbe Referenzpanel;
Vergleich des Fingerabdrucks jedes Kandidaten mit demjenigen des Aktivators; und
Identifizieren einer Verbindung, deren Fingerabdruck demjenigen des Aktivators ähnelt, als erfolgreichen Kandidaten.

15. Verbindung der Formel:

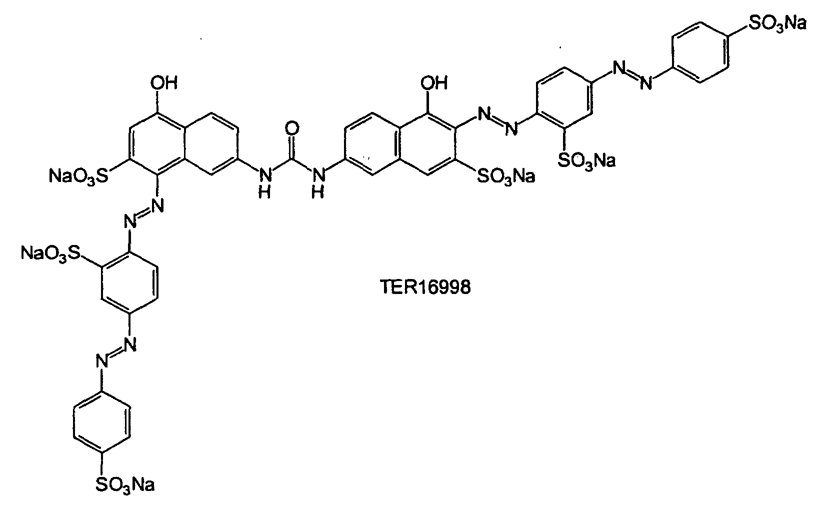

16. Zusammensetzung, umfassend die Verbindung gemäss Anspruch 15 oder ein pharmazeutisch akzeptables Salz davon und einen pharmazeutisch akzeptablen Träger.

## Revendications

1. Méthode in vitro pour moduler l'activité de kinase du récepteur d'insuline et/ou pour potentialiser l'activation par l'insuline du récepteur d'insuline et/ou pour potentialiser la stimulation par l'insuline de l'absorption du glucose cellulaire ou pour stimuler l'absorption du glucose dans les cellules présentant le récepteur d'insuline, méthode qui comprend la mise en contact dudit récepteur d'insuline ou de sa partie kinase ou bien desdites cellules avec un composé qui comprend la formule 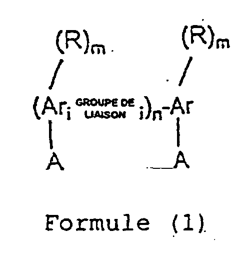 dans laquelle
chaque groupe Ar représente indépendamment un groupement aromatique choisi entre des groupements benzène, naphtalène, pyridine, quinoléine et benzothiazble ;
chaque groupe A représente indépendamment un groupe -SO₃X, OP(OX)₃ ou -COOX, dans lequel X représente un atome d'hydrogène ou un cation ;
chaque groupe R représente indépendamment un groupement hydrocarbyle substitué ou non substitué, ramifié ou non ramifié, cyclique, aromatique ou non aromatique, dans lequel les chaînes hydrocarbyle non ramifiées peuvent être interrompues par un ou plusieurs hétéroatomes de O, N ou S ; ou bien chaque groupe R représente indépendamment un groupe OR', NR'₂ ou SR' dans lequel R' représente H ou un groupe R répondant à la définition précitée ;
m est égal à 0, 1 ou 2 ; n a une valeur de 1 à 6 ; et
chaque groupe de liaison représente indépendamment un groupe -CH₂-, -N=N-, -CH=CH-, -NHCO- ou -NHCONH- ou un de ses isostères, dans lequel, lorsque n est égal à 1, au moins un groupe Ar doit comprendre au moins 2 noyaux aromatiques condensés.

2. Méthode suivant la revendication 1, dans laquelle le composé de formule (1) répond à la formule 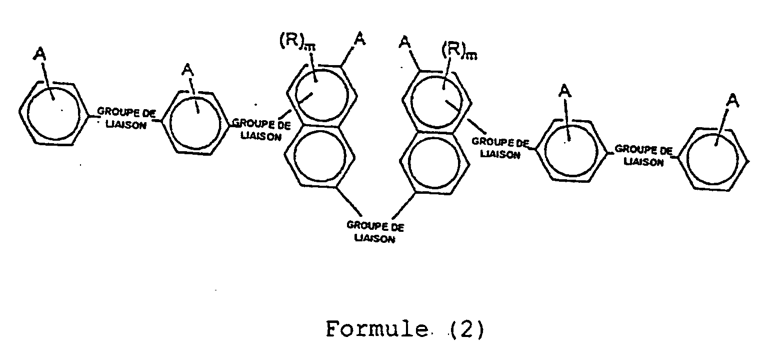 dans laquelle chacun des symboles R, A, "groupe de liaison" et m répond à la définition suivant la revendication 1.

3. Méthode suivant la revendication 1, dans laquelle le composé de formule (1) répond à la formule 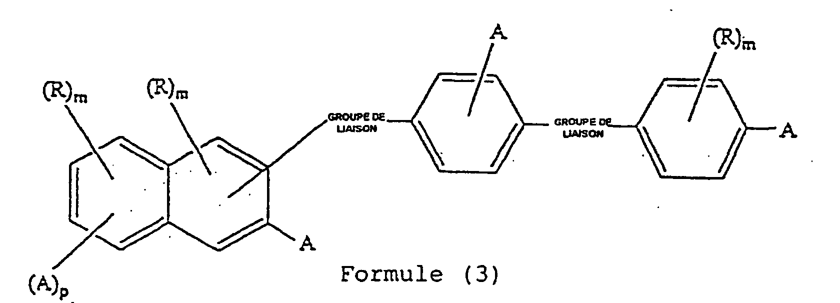 dans laquelle chacun des symboles R, A, "groupe de liaison" et m répond à la définition suivant la revendication 1 et dans laquelle p est égal à 0 ou 1.

4. Méthode suivant la revendication 1, dans laquelle m est égal à 0 ou 1, n a une valeur de 4 à 6 et chaque groupe de liaison représente indépendamment un groupe -CH₂-,CH=CH- ou -NHCO- ou un de ses isostères.

5. Méthode suivant la revendication 1, dans laquelle ledit composé répond à la formule 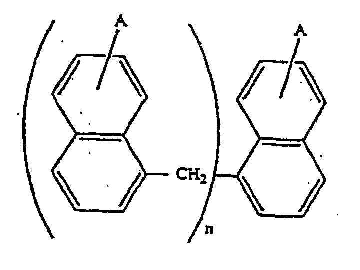 dans laquelle n a une valeur de 4 à 6 et A répond à la définition suivant la revendication 1.

6. Méthode suivant la revendication 2 ou 3, dans laquelle chaque groupe R représente indépendamment un groupe OH ou 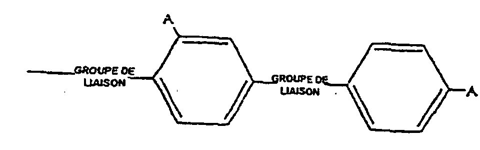 ou 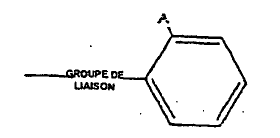 dans lequel A et le groupe de liaison répondent aux définitions suivant la revendication 1.

7. Méthode suivant la revendication 2 ou 3, dans laquelle m est égal à 0 ou 1 et chaque groupe R représente indépendamment un groupe OH.

8. Méthode suivant la revendication 1 ou 4, dans laquelle chaque groupe R représente indépendamment un groupe alkyle en C₁ à C₆.

9. Méthode suivant l'une quelconque des revendications 1 à 8, dans laquelle chaque groupe A représente indépendamment un groupe -SO₃X ou -COOX, dans lequel X représente H ou un cation.

10. Méthode suivant l'une quelconque des revendications 1 à 9, dans laquelle tous les indices m sont égaux à 0.

11. Méthode suivant la revendication 3, dans laquelle ledit composé répond à la formule 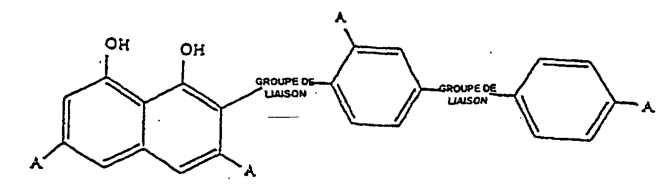 dans laquelle chaque groupe de liaison représente indépendamment un groupe -NHCO- ou -CH=CH-, et chaque groupe A représente indépendamment un groupe -SO₃X ou -COOX dans lequel X représente H ou un cation.

12. Méthode suivant la revendication 2, dans laquelle le composé est choisi dans le groupe consistant en 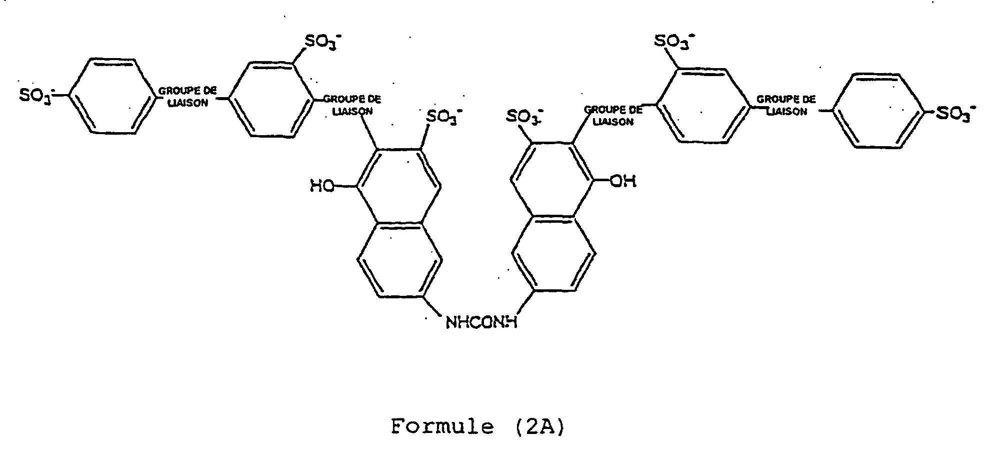 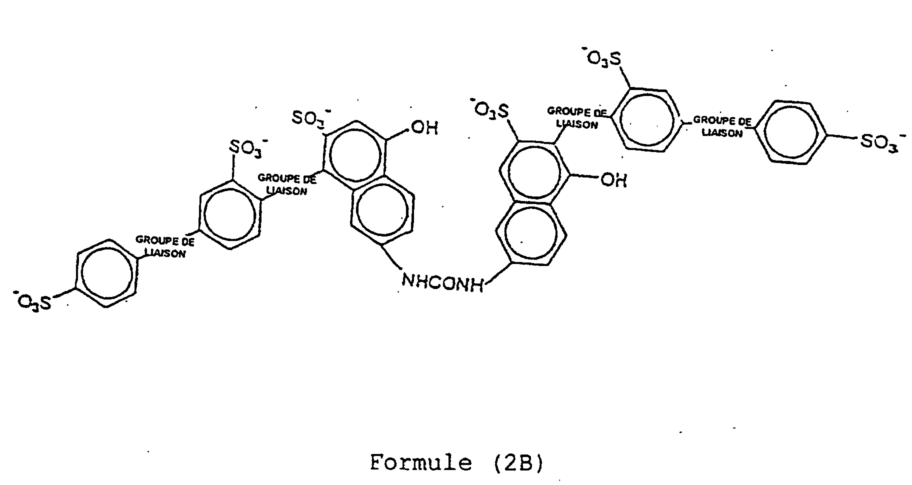 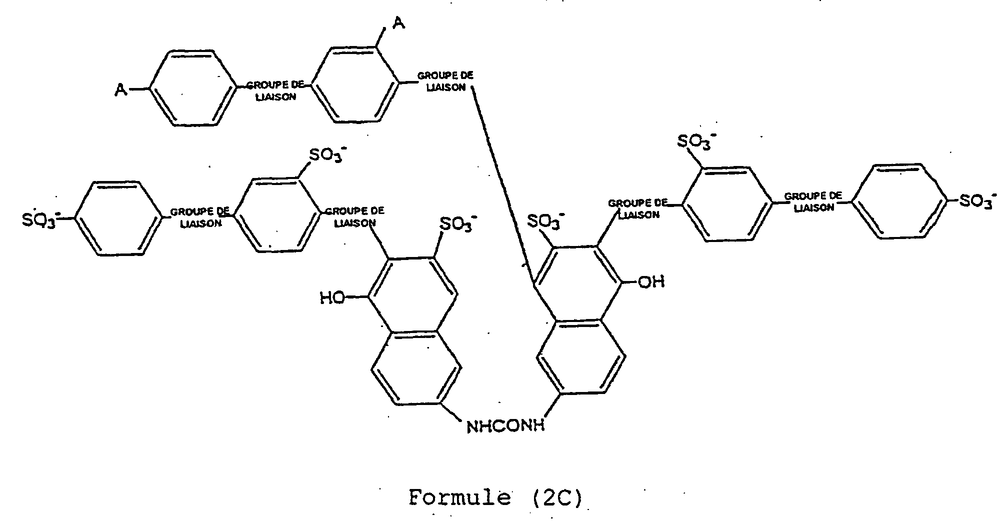 formules dans lesquelles chaque groupe de liaison représente indépendamment un groupe -N=N- ou -NHCO-.

13. Méthode pour concevoir et synthétiser une molécule qui active le récepteur d'insuline, méthode qui comprend :
la détermination d'un activateur qui est le composé suivant l'une quelconque des revendications 1 à 12 pour des caractéristiques structurales qui présentent une corrélation avec lesdites activités ; la synthèse d'un composé contenant ces caractéristiques structurales ; et le test dudit composé pour son aptitude à activer le récepteur d'insuline afin de le vérifier en tant qu'activateur.

14. Méthode pour sélectionner les composés candidats pour l'aptitude à activer le récepteur d'insuline, méthode qui comprend :
l'obtention d'une empreinte de chaque candidat par rapport à une gamme de référence ;
l'obtention d'une empreinte d'un activateur qui est le composé suivant l'une quelconque des revendications 1 à 12 par rapport à cette même gamme de référence ;
la comparaison de l'empreinte de chaque candidat avec celle dudit activateur ; et
l'identification, comme candidat couronné de succès, d'un composé dont l'empreinte ressemble à celle dudit activateur.

15. Composé de formule

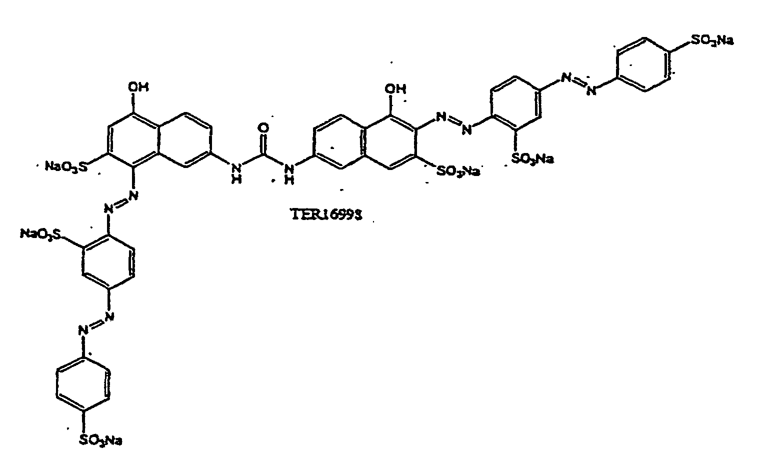

16. Composition comprenant le composé suivant la revendication 15 ou un de ses sels pharmaceutiquement acceptables et un excipient pharmaceutiquement acceptable.
